# EUROPEAN PATENT APPLICATION

(11) **EP 4 752 161 A1**
(43) Date of publication of application: **03.06.2026**
(21) Application number: 24844804.5
(22) Date of filing: 24.07.2024
(51) Int. Cl.: C07K 19/00, C12N 15/62, C07K 14/725, C12N 5/10, C12N 15/86, A61P 35/00, A61P 37/00

(54) **FUSION PROTEIN FOR TREATING DISEASES RELATED TO CD20**

(30) Priority: 25.07.2023 WO PCT/CN2023/109110
(71) Applicant: JW Therapeutics R&D (Shanghai) Co., Ltd., Shanghai 201210 (CN); JW Therapeutics (Shanghai) Co., Ltd., Shanghai 201210 (CN)
(72) Inventor: GU, Haihua, Shanghai 201210 (CN); GUO, Jun, Shanghai 201210 (CN); CORDOBA, Shaun, Shanghai 201210 (CN)
(74) Representative: Sagittarius IP
(86) International application number: PCT/CN2024/107288
(87) International publication number: WO 2025/021113

(57) **Abstract**

The present application provides fusion proteins e.g. CARs directing against CD20, and their use in the treatment of diseases.

## Description

### CROSS-REFERENCE TO RELATED APPLICATIONS

This application is based on an international patent applications with the application number PCT/CN2023/109110, and claims priority from said international patent applications, the entire content of which is hereby incorporated by reference into this application.

### FIELD OF THE INVENTION

The instant disclosure relates generally to fusion proteins, e.g. CARs that specifically target CD20. It further provides immunoresponsive cells comprising such CAR, and methods of using such CARs and such cells for treating cancers and autoimmune diseases.

### BACKGROUND

Chimeric antigen receptors (CARs) are artificial molecules that redirect the specificity of T cells to antigens or their epitopes, which are diverse in different diseases. A CAR construct usually comprises an extracellular antigen-binding domain, a spacer, a transmembrane domain and an intracellular signaling domain. Due to the complexity of CAR structures, it requires optimization depending on antigens. Thus, there is a need for optimal CARs for specific antigens.

### BRIEF SUMMARY

The present disclosure provides novel fusion proteins with optimal properties for cell therapy. Specifically, the disclosure relates to CARs having particular structures that allow the CARs to position the antigen-binding domain at a distance from a target epitope such that the CAR can effectively interact with the target cell.

In one aspect, the disclosure provides a fusion protein comprising an extracellular antigen-binding domain, a transmembrane domain, a spacer located between the extracellular antigen-binding domain and the transmembrane domain and an intracellular domain, wherein: a) the extracellular antigen-binding domain is capable of specifically binding to the same epitope on CD20 as a reference antibody that comprises the amino acid sequence of SEQ ID NO: 1, SEQ ID NO: 2 or SEQ ID NO: 3; b) the spacer consists of a hinge domain from CD28 or a hinge domain from an immunoglobulin, wherein the spacer consisting of the hinge domain from the immunoglobulin is at 12, 13, 14 or 15 amino acids in length; and c) the transmembrane domain comprises a transmembrane domain from CD28.

In one aspect, the disclosure provides a fusion protein comprising an extracellular antigen-binding domain, a transmembrane domain, a spacer located between the extracellular antigen-binding domain and the transmembrane domain and an intracellular signaling domain, wherein: a) the extracellular antigen-binding domain is capable of specifically binding to the same epitope on CD20 as a reference antibody that comprises the amino acid sequence of SEQ ID NO: 1, SEQ ID NO: 2 or SEQ ID NO: 3; b) the spacer consists of a hinge domain from CD28 or a hinge domain from an immunoglobulin, wherein the spacer consisting of the hinge domain from the immunoglobulin is at 12, 13, 14 or 15 amino acids in length ; c) the transmembrane domain comprises a transmembrane domain from CD28; and d) the intracellular signaling domain comprises a primary intracellular signaling domain and a co-stimulatory signaling domain located between the transmembrane domain and the primary intracellular signaling domain, wherein the co-stimulatory signaling domain comprises a signaling domain of CD137, the primary intracellular signaling domain comprises a cytoplasmic signaling domain of CD3ζ.

In one aspect, the extracellular antigen-binding domain comprises a heavy chain variable region (VH) and a light chain variable region (VL). In one aspect, the VH comprises a HCDR1, a HCDR2 and a HCDR3 comprising the amino acid sequences of SEQ ID NO: 6, SEQ ID NO: 7 and SEQ ID NO: 8, respectively; the VL comprises a LCDR1, a LCDR2 and a LCDR3 comprising the amino acid sequences of SEQ ID NO: 9, SEQ ID NO: 10 and SEQ ID NO: 11, respectively. In one aspect, the VH comprises a HCDR1, a HCDR2 and a HCDR3 comprising the amino acid sequences of SEQ ID NO: 14, SEQ ID NO: 15 and SEQ ID NO: 16, respectively; the VL comprises a LCDR1, a LCDR2 and a LCDR3 comprising the amino acid sequences of SEQ ID NO: 17, SEQ ID NO: 18 and SEQ ID NO: 19, respectively. In one aspect, the VH comprises a HCDR1, a HCDR2 and a HCDR3 comprising the amino acid sequences of SEQ ID NO: 22, SEQ ID NO: 23 and SEQ ID NO: 24, respectively; the VL comprises a LCDR1, a LCDR2 and a LCDR3 comprising the amino acid sequences of SEQ ID NO: 25, SEQ ID NO: 26 and SEQ ID NO: 27, respectively. In one aspect, the VH comprises an amino acid sequence having at least 90% sequence identity to SEQ ID NO: 4; the VL comprises an amino acid sequence having at least 90% sequence identity to SEQ ID NO: 5. In one aspect, the VH comprises an amino acid sequence having at least 90% sequence identity to SEQ ID NO: 12; the VL comprises an amino acid sequence having at least 90% sequence identity to SEQ ID NO: 13. In one aspect, the VH comprises an amino acid sequence having at least 90% sequence identity to SEQ ID NO: 20; the VL comprises an amino acid sequence having at least 90% sequence identity to SEQ ID NO: 21. In one aspect, the VH comprises the amino acid sequence of SEQ ID NO: 4; the VL comprises the amino acid sequence of SEQ ID NO: 5. In one aspect, the VH comprises the amino acid sequence of SEQ ID NO: 12; the VL comprises the amino acid sequence of SEQ ID NO: 13. In one aspect, the VH comprises the amino acid sequence of SEQ ID NO: 20; the VL comprises the amino acid sequence of SEQ ID NO: 21.

In one aspect, the extracellular antigen-binding domain is an scFv. In one aspect, the VH is fused by its C-terminus via a flexible polypeptide linker to the N-terminus of the VL. In one aspect, the extracellular antigen-binding domain comprises the amino acid sequence of SEQ ID NO: 1, SEQ ID NO: 2 or SEQ ID NO: 3.

In one aspect, the spacer consists of the hinge domain from CD28. In one aspect, the spacer consists of an amino acid sequence having at least 90% sequence identity to SEQ ID NO: 30. In one aspect, the spacer consists of the amino acid sequence of SEQ ID NO: 30.

In one aspect, the spacer consists of the hinge domain from IgG4, wherein the spacer consisting of the hinge domain from IgG4 is at 12 amino acids in length. In one aspect, the spacer consists of an amino acid sequence having at least 90% sequence identity to SEQ ID NO: 31. In one aspect, the spacer consists of the amino acid sequence of SEQ ID NO: 31.

In one aspect, the transmembrane domain comprises an amino acid sequence having at least 90% sequence identity to SEQ ID NO: 33. In one aspect, the transmembrane domain comprises the amino acid sequence of SEQ ID NO: 33.

In one aspect, the intracellular signaling domain comprises a primary intracellular signaling domain. In one aspect, the primary intracellular signaling domain comprises a cytoplasmic signaling domain of CD3ζ. In one aspect, the primary intracellular signaling domain comprises an amino acid sequence having at least 90% sequence identity to SEQ ID NO: 35. In one aspect, the primary intracellular signaling domain comprises the amino acid sequence of SEQ ID NO: 35.

In one aspect, the intracellular signaling domain comprises a co-stimulatory signaling domain located between the transmembrane domain and the primary intracellular signaling domain. In one aspect, the co-stimulatory signaling domain comprises a signaling domain of protein selected from the group consisting of CD27, CD28, CD137, OX40, CD30, CD40, CD3, HVEM, ICOS, Myd88, LFA-1, ICOS, CD2, CD7, NKG2C, B7-H3, Ligands of CD83 and a combination thereof. In one aspect, the co-stimulatory signaling domain comprises a signaling domain of CD137. In one aspect, the co-stimulatory signaling domain comprises an amino acid sequence having at least 90% sequence identity to SEQ ID NO: 34. In one aspect, the co-stimulatory signaling domain comprises the amino acid sequence of SEQ ID NO: 34.

In one aspect, a) the extracellular antigen-binding domain is capable of specifically binding to the same epitope on CD20 as a reference antibody that comprises the amino acid sequence of SEQ ID NO: 1; b) the spacer consists of a hinge domain from CD28; c) the transmembrane domain comprises a transmembrane domain from CD28; and d) the intracellular signaling domain comprises a signaling domain of CD137 and a cytoplasmic signaling domain of CD3ζ. In one aspect, a) the extracellular antigen-binding domain comprises a VH comprising a HCDR1, a HCDR2 and a HCDR3 comprising the amino acid sequences of SEQ ID NO: 6, SEQ ID NO: 7 and SEQ ID NO: 8, respectively, and a VL comprising a LCDR1, a LCDR2 and a LCDR3 comprising the amino acid sequences of SEQ ID NO: 9, SEQ ID NO: 10 and SEQ ID NO: 11, respectively; b) the spacer consists of the amino acid sequence of SEQ ID NO: 30; c) the transmembrane domain comprises the amino acid sequence of SEQ ID NO: 33; and d) the intracellular signaling domain comprises the amino acid sequences of SEQ ID NO: 34 and SEQ ID NO: 35. In one aspect, the fusion protein comprises the amino acid sequence of SEQ ID NO: 36.

In one aspect, a) the extracellular antigen-binding domain is capable of specifically binding to the same epitope on CD20 as a reference antibody that comprises the amino acid sequence of SEQ ID NO: 1; b) the spacer consists of a hinge domain from the IgG4, wherein the spacer consisting of the hinge domain from IgG4 is at 12 amino acids in length; c) the transmembrane domain comprises a transmembrane domain from CD28; d) the intracellular signaling domain comprises a signaling domain of CD137 and a cytoplasmic signaling domain of CD3ζ. In one aspect, a) the extracellular antigen-binding domain comprises a VH comprising a HCDR1, a HCDR2 and a HCDR3 comprising the amino acid sequences of SEQ ID NO: 6, SEQ ID NO: 7 and SEQ ID NO: 8, respectively, and a VL comprising a LCDR1, a LCDR2 and a LCDR3 comprising the amino acid sequences of SEQ ID NO: 9, SEQ ID NO: 10 and SEQ ID NO: 11, respectively; b) the spacer consists of the amino acid sequence of SEQ ID NO: 31; c) the transmembrane domain comprises the amino acid sequence of SEQ ID NO: 33; and d) the intracellular signaling domain comprises the amino acid sequences of SEQ ID NO: 34 and SEQ ID NO: 35. In one aspect, the fusion protein comprises the amino acid sequence of SEQ ID NO: 38.

In one aspect, a) the extracellular antigen-binding domain is capable of specifically binding to the same epitope on CD20 as a reference antibody that comprises the amino acid sequence of SEQ ID NO: 2; b) the spacer consists of a hinge domain from the IgG4, wherein the spacer consisting of the hinge domain from IgG4 is at 12 amino acids in length; c) the transmembrane domain comprises a transmembrane domain from CD28; d) the intracellular signaling domain comprises a signaling domain of CD137 and a cytoplasmic signaling domain of CD3ζ. In one aspect, a) the extracellular antigen-binding domain comprises a VH comprising a HCDR1, a HCDR2 and a HCDR3 comprising the amino acid sequences of SEQ ID NO: 14, SEQ ID NO: 15 and SEQ ID NO: 16, respectively, and a VL comprising a LCDR1, a LCDR2 and a LCDR3 comprising the amino acid sequences of SEQ ID NO: 17, SEQ ID NO: 18 and SEQ ID NO: 19, respectively; b) the spacer consists of the amino acid sequence of SEQ ID NO: 31; c) the transmembrane domain comprises the amino acid sequence of SEQ ID NO: 33; and d) the intracellular signaling domain comprises the amino acid sequences of SEQ ID NO: 34 and SEQ ID NO: 35. In one aspect, the fusion protein comprises the amino acid sequence of SEQ ID NO: 37.

The disclosure further provides an isolated nucleic acid encoding the fusion protein as described herein.

The disclosure further provides a vector comprising the nucleic acid as described herein. In one aspect, the vector is a viral vector. In one aspect, the viral vector is a retroviral, lentiviral, adenoviral, or adeno-associated viral vector.

The disclosure further provides a cell comprising the fusion protein, the isolated nucleic acid, or the vector as described herein. In one aspect, the cell is a lymphocyte. In one aspect, the cell is a NK cell or a T cell.

The disclosure further provides a pharmaceutical formulation comprising a fusion protein or the cell as described herein and a pharmaceutically acceptable carrier.

The disclosure provides a method of treating a disease or disorder associated with CD20 in a subject, comprising administering the fusion protein, the isolated nucleic acid, the vector, the cell or the pharmaceutical formulation as described herein to the subject. In one aspect, the subject has a relapsed or refractory disease or disorder associated with CD20 or has been previously treated with at least one regimen comprising a medicament directing against CD20. Also provided is the use of the fusion protein, the isolated nucleic acid, the vector, the cell or the pharmaceutical formulation as described herein in the manufacture of a medicament for the treatment of a disease or disorder associated with CD20. The disclosure further provides the fusion protein, the isolated nucleic acid, the vector, the cell or the pharmaceutical formulation as described herein for use as a medicament. In one aspect, the disease or disorder is a cancer or an autoimmune disease. In one aspect, the cancer is selected from the group consisting of leukemia, lymphoma, lung cancer, liver cancer, melanoma, breast cancer, prostate cancer, colon cancer, renal cell carcinoma, ovarian cancer, neuroblastoma and rhabdomyosarcoma. In one aspect, the cancer is leukemia or lymphoma. In one aspect, the autoimmune disease is selected from the group consisting of rheumatoid arthritis, systemic lupus erythematosus, multiple sclerosis, Wegener's disease, inflammatory bowel disease, ulcerative colitis, idiopathic thrombocytopenic purpura, thrombotic thrombocytopenic purpura, autoimmune thrombocytopenia, psoriasis, IgA nephropathy, IgM polyneuropathies, myasthenia gravis, ANCA associated vasculitis, diabetes mellitus, Reynaud's syndrome, Sjogren's syndrome, Neuromyelitis Optica and glomerulonephritis.

### BRIEF DESCRIPTION OF THE DRAWINGS

Fig. 1A shows the degree of CD20 expression of different target cells. Fig. 1B depicts the result for an activation assay in Jurkat cells.
Fig. 2A to 2C show the percentage of CD25, CD69 and Ki67 positive populations in primary T cells after stimulation by either CD20^{KO}, CD20^{lo} or CD20^{WT} Raji cells for 48 hours.
Fig. 3A to 3B show the IFN-γ and IL-2 secreted by T cells transduced with CARs after stimulation by different target cell lines.
Fig. 4 shows the 24-hour killing score of T cells on different target cell lines.

### DETAILED DESCRIPTION

### I. Definitions

Unless defined otherwise, all technical and scientific terms used herein have the same meaning as commonly understood by one of ordinary skill in the art to which the invention pertains.

The term "a" and "an" refers to one or to more than one (i.e., to at least one) of the grammatical object of the article. By way of example, "a module" means one module or more than one module.

The terms "comprising", "comprises" as used herein are synonymous with "including", "includes" or "containing", "contains", and are inclusive or open-ended and do not exclude additional, non-recited members, elements or method steps. The terms "comprising", "comprises" and the like also include the term "consisting of".

The term "antibody" herein is used in the broadest sense and encompasses various antibody structures, including but not limited to monoclonal antibodies, polyclonal antibodies, multispecific antibodies (e.g. bispecific antibodies), intact antibody and antibody fragments so long as they exhibit the desired antigen-binding activity.

The terms "full-length antibody," "intact antibody," and "whole antibody" are used herein interchangeably to refer to an antibody having a structure substantially similar to a native antibody structure. For example, an intact antibody of IgG class comprises two light chains and two heavy chains that are disulfide-bonded. From N- to C-terminus, each heavy chain has a variable domain (VH), followed by three constant domains (CH1, CH2, and CH3), also called a heavy chain constant region. Similarly, from N- to C-terminus, each light chain has a variable domain (VL), followed by a constant light (CL) domain, also called a light chain constant region. The heavy chain of an antibody may be assigned to one of five types, called IgA, IgD, IgE, IgG, or IgM, some of which may be further divided into subtypes, e.g. IgG1, IgG2, IgG3, IgG4, IgA1 and IgA2. The light chain of an antibody may be assigned to one of two types, called kappa (K) and lambda (λ), based on the amino acid sequence of its constant domain.

The "antibody fragment" refers to a molecule other than an intact antibody that comprises a portion of an intact antibody that binds the antigen. Examples of antibody fragments include but are not limited to Fv, Fab, Fab', Fab'-SH, F(ab')₂, diabodies, linear antibodies, single-chain antibody molecules (e.g. scFv and scFab), single-domain antibodies, and multispecific antibodies formed from antibody fragments.

A "Single-chain Fv" or "scFv" is a fusion protein of the variable regions of the light (VL) and heavy chain (VH), connected with a short linker peptide of about ten to 25 amino acids. The linker is usually flexible and can either connect the N-terminus of the VH with the C-terminus of the VL, or vice versa. This protein retains the specificity of the original antibody, despite removal of the constant regions and the introduction of the linker.

The term "antigen binding domain" refers to the part of an antibody that comprises the area which binds to and is complementary to part or all of an antigen. An antigen binding domain may be provided by, for example, one or more antibody variable domains (also called antibody variable regions). In preferred aspects, an antigen binding domain comprises an antibody light chain variable domain (VL) and an antibody heavy chain variable domain (VH).

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three complementarity determining regions (CDRs). In some instances, a single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. As used herein in connection with variable region sequences, "Kabat numbering" refers to the numbering system set forth by Kabat et al., Sequences of Proteins of Immunological Interest, 5th Ed. Public Health Service, National Institutes of Health, Bethesda, MD (1991).

The terms "complementarity determining region" and "CDR" are known to refer to non-contiguous sequences of amino acids within antibody variable regions, which confer binding specificity and/or binding affinity to antigens. In general, there are three CDRs in each heavy chain variable region (HCDR1, HCDR2, HCDR3) and three CDRs in each light chain variable region (LCDR1, LCDR2, LCDR3). "Framework regions" and "FR" refer to the non-CDR portions of the variable regions of the heavy and light chains. Table 1, below, lists exemplary position boundaries of HCDR1, HCDR2, HCDR3, LCDR1, LCDR2 and LCDR3 as identified by Kabat, Chothia, AbM, IMGT and Contact schemes, respectively. Unless otherwise indicated, the amino acid sequences of CDRs shown in the present disclosure are determined according to Kabat.

**Table 1. The CDR regions according to various numbering systems**

| CDR | Kabat | AbM | Chothia | IMGT | Contact |
|---|---|---|---|---|---|
| HCDR1 | 31-35B | 26-35 | 26-32 | 27-38 | 30-35 |
| HCDR2 | 50-65 | 50-58 | 52-56 | 56-65 | 47-58 |
| HCDR3 | 95-102 | 95-102 | 95-102 | 105-117 | 93-101 |
| LCDR1 | 24-34 | 24-34 | 24-34 | 27-38 | 30-36 |
| LCDR2 | 50-56 | 50-56 | 50-56 | 56-65 | 46-55 |
| LCDR3 | 89-97 | 89-97 | 89-97 | 105-117 | 89-96 |

The term "specifically binds" or "bind", which can be used interchangeably, as used herein means the ability of a protein, under specifically binding conditions, to bind to a target protein such that its affinity or avidity is at least 5 times as great, but optionally at least 10, 20, 30, 40, 50, 100, 250 or 500 times as great, or even at least 1000 times as great as the average affinity or avidity of the same protein to a collection of random peptides or polypeptides of sufficient statistical size. A specifically binding protein does not necessarily bind exclusively to a single target molecule, but may also specifically bind to a non-target molecule if similarity in structural conformation exists between the target and non-target (e.g., paralogs or orthologs). One skilled artisan recognizes that specifically binding to a molecule having the same function in a different species of animal or to a non-target molecule having a substantially similar epitope as the target molecule is possible, and this does not detract from the specificity of binding, which is determined relative to a statistically valid collection of unique non-targets. Thus, a polypeptide may specifically bind to more than one distinct species of target molecule due to cross-reactivity.

The term "epitope" refers to the moieties of an antigen that specifically interact with an antibody. Such moieties, referred to herein as epitopic determinants, typically comprise, or are part of, elements such as amino acid side chains or sugar side chains. An epitopic determinant can be defined by methods known in the art, e.g. by crystallography or by hydrogen-deuterium exchange. At least one or some of the moieties on the antibody molecule that specifically interact with an epitopic determinant are typically located in a CDR(s). Typically, an epitope has a specific three-dimensional structural characteristic and/or a specific charge characteristic. Some epitopes are linear epitopes while others are conformational epitopes.

The term "hinge" as used herein refers to a flexible polypeptide connector region providing structural flexibility and spacing to the connected flanking polypeptide regions and can consist of natural or synthetic polypeptides.

"Percent (%) amino acid sequence identity" and "homology" with respect to a peptide, polypeptide or antibody sequence are defined as the percentage of amino acid residues in a candidate sequence that are identical with the amino acid residues in the specific peptide or polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or MEGALIGNTM (DNASTAR) software. Those skilled in the art can determine appropriate parameters for measuring alignment, including any algorithms needed to achieve maximal alignment over the full length of the sequences being compared.

The term "fusion protein" refers to a hybrid (e.g., chimeric, recombinant) polypeptide which comprises protein domains from at least two different proteins.

The term "Chimeric Antigen Receptor" or alternatively a "CAR" refers to a fusion protein engineered to contain two or more naturally occurring or engineered amino acid sequences linked together in a way that does not occur naturally in a host cell, which fusion protein can function as a receptor when present on a surface of a cell. CARs of the present disclosure include an extracellular portion comprising an antigen-binding domain (e.g. an scFv) linked to a spacer sequence, a transmembrane domain and one or more intracellular signaling domains (optionally containing co-stimulatory domain(s)).

The term "extracellular domain" or "ectodomain" which can be used interchangeably, as used herein refers to the region of a membrane protein, such as a transmembrane protein, that lies outside the vesicular membrane. Ectodomains often comprise binding domains that specifically bind to ligands or cell surface receptors. The term "extracellular antigen-binding domain" refers to an extracellular domain or a part of extracellular domain that is capable of specifically binding to an antigen.

The term "endodomain" or "intracellular domain," or "cytoplasmic domain" which can be used interchangeably, as used herein refers to the region found in some membrane proteins, such as transmembrane proteins, which extends into the interior space defined by the cell surface membrane. In some cells, the endodomain interacts with intracellular constituents and can play a role in signal transduction and thus, in some cases, can be an intracellular signaling domain.

The term "transmembrane domain" as used herein means a domain found in a membrane protein that substantially or completely spans a lipid bilayer such as those lipid bilayers found in biological membranes, such as those in a mammalian cell, or in an artificial construct such as a liposome. A transmembrane protein can pass through both layers of the lipid bilayer once or multiple times.

The term "spacer" as used herein refers to a polypeptide sequence (e.g. a hinge) which is capable of covalently linking together two spaced moieties: an antigen-binding domain, and the transmembrane domain of the fusion protein (e.g. a CAR).

The term "flexible polypeptide linker" as used herein refers to a peptide which joins other peptides to form a functional protein. In the context of an scFv, the flexible polypeptide linker refers to a peptide linker which joins the VH and VL to form an scFv specifically binding to an antigen.

The term "expression" refers to the process by which a polypeptide is produced based on the encoding sequence of a nucleic acid molecule, such as a gene. The process may include transcription, post-transcriptional control, post-transcriptional modification, translation, post-translational control, post-translational modification, or any combination thereof.

The term "encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (e.g., rRNA, tRNA and mRNA) or a defined sequence of amino acids and the biological properties resulting therefrom. Thus, a gene, cDNA, or RNA, encodes a protein if transcription and translation of mRNA corresponding to that gene produces the protein in a cell or other biological system.

The terms "polypeptide", "peptide" and "protein" are used interchangeably, and refer to a compound comprised of amino acid residues covalently linked by peptide bonds. A polypeptide must contain at least two amino acids, and no limitation is placed on the maximum number of amino acids that can be comprised in a peptide's sequence. The term "polypeptide" is also intended to refer to the products of post-expression modifications of the polypeptide, including without limitation glycosylation, acetylation, phosphorylation, amidation, derivatization by known protecting/blocking groups, proteolytic cleavage, or modification by non-naturally occurring amino acids. A polypeptide of the disclosure may be of a size of about 3 or more, 5 or more, 10 or more, 20 or more, 25 or more, 50 or more, 75 or more, 100 or more, 200 or more, 500 or more, 1,000 or more, or 2,000 or more amino acids. Polypeptides may have a defined three-dimensional structure, although they do not necessarily have such structure.

An "isolated" nucleic acid molecule refers to a nucleic acid molecule that has been separated from a component of its natural environment. An isolated nucleic acid molecule includes a nucleic acid molecule contained in cells that ordinarily contain the nucleic acid molecule, but the nucleic acid molecule is present extrachromosomally or at a chromosomal location that is different from its natural chromosomal location. "Isolated polynucleotide (or nucleic acid) encoding a fusion protein" refers to one or more polynucleotide molecules encoding the fusion protein, including such polynucleotide molecule(s) in a single vector or separate vectors, and such polynucleotide molecule(s) present at one or more locations in a host cell.

The term "vector", as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked.

The terms "host cell", "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages.

The term "subject" or "individual" is intended to include living organisms in which an immune response can be elicited (e.g., mammals, human).

As used herein, "treatment" (and grammatical variations thereof such as "treat" or "treating") refers to clinical intervention in an attempt to alter the natural course of a disease in the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis.

The term "pharmaceutical composition" refers to a preparation which is in such form as to permit the biological activity of an active ingredient contained therein to be effective, and which contains no additional components which are unacceptably toxic to a subject to which the preparation would be administered. A pharmaceutical composition usually comprises one or more pharmaceutically acceptable carrier(s). A "pharmaceutically acceptable carrier" refers to an ingredient in a pharmaceutical composition, other than an active ingredient, which is nontoxic to a subject. A pharmaceutically acceptable carrier includes, but is not limited to, a buffer, excipient, stabilizer, or preservative.

An "effective amount" of an agent, e.g., a pharmaceutical composition, refers to an amount effective, at dosages and for periods of time necessary, to achieve the desired therapeutic or prophylactic result.

### II. Fusion protein

The present disclosure provides a novel fusion protein with optimal properties such as producibility, stability, binding affinity, biological activity, targeting efficiency, reduced toxicity, an extended dosage range that can be given to a patient and thereby a possibly enhanced efficacy. Herein provided is a fusion protein comprising a) an extracellular antigen-binding domain; b) a spacer; c) a transmembrane domain; and d) an intracellular signaling domain. In some aspects, the fusion protein is a CAR.

### 2.1 Extracellular antigen-binding domain

In one aspect, provided is the fusion protein as defined herein, wherein the extracellular antigen-binding domain comprises one or more antibodies. In one aspect, the antibody is Fv, Fab, Fab', Fab'-SH, F(ab')₂, diabody, linear antibody, single-chain antibody molecule (e.g. scFv and scFab), single-domain antibody, or multispecific antibody formed from antibody fragments. In a particular embodiment, the extracellular antigen-binding domain comprises an scFv.

In one aspect, the extracellular antigen binding domain is capable of binding to CD20. In one aspect, the fusion protein is capable of binding to the same epitope on CD20 as any anti-CD20 antibody in the art (e.g. Ofatumumab, Obinutuzumab, Rituximab, Ocrelizumab, Veltuzumab, Ibritumomab, Tositumumab, Odronextamab, Ublituximab, Leu16, IF5, 1.5.3). In one aspect, the extracellular antigen-binding domain is capable of binding to the same epitope on CD20 as a reference antibody that comprises the amino acid sequence of SEQ ID NO: 1, SEQ ID NO: 2 or SEQ ID NO: 3.

In one aspect, the extracellular antigen-binding domain comprises a VH and a VL. In one aspect, the VH comprises a HCDR1, a HCDR2 and a HCDR3 comprised in SEQ ID NO: 4, the VL comprises a LCDR1, a LCDR2 and a LCDR3 comprised in SEQ ID NO: 5. In one aspect, the VH comprises a HCDR1, a HCDR2 and a HCDR3 comprised in SEQ ID NO: 12, the VL comprises a LCDR1, a LCDR2 and a LCDR3 comprised in SEQ ID NO: 13. In one aspect, the VH comprises a HCDR1, a HCDR2 and a HCDR3 comprised in SEQ ID NO: 20, the VL comprises a LCDR1, a LCDR2 and a LCDR3 comprised in SEQ ID NO: 21.

In one aspect, the VH comprises a HCDR1, a HCDR2 and a HCDR3 comprising or consisting of the amino acid sequences of SEQ ID NO: 6, SEQ ID NO: 7 and SEQ ID NO: 8, respectively; the VL comprises a LCDR1, a LCDR2 and a LCDR3 comprising or consisting of the amino acid sequences of SEQ ID NO: 9, SEQ ID NO: 10 and SEQ ID NO: 11, respectively. In one aspect, the VH comprises a HCDR1, a HCDR2 and a HCDR3 comprising or consisting of the amino acid sequences of SEQ ID NO: 14, SEQ ID NO: 15 and SEQ ID NO: 16, respectively; the VL comprises a LCDR1, a LCDR2 and a LCDR3 comprising or consisting of the amino acid sequences of SEQ ID NO: 17, SEQ ID NO: 18 and SEQ ID NO: 19, respectively. In one aspect, the VH comprises a HCDR1, a HCDR2 and a HCDR3 comprising or consisting of the amino acid sequences of SEQ ID NO: 22, SEQ ID NO: 23 and SEQ ID NO: 24, respectively; the VL comprises a LCDR1, a LCDR2 and a LCDR3 comprising or consisting of the amino acid sequences of SEQ ID NO: 25, SEQ ID NO: 26 and SEQ ID NO: 27, respectively.

In one aspect, the VH comprises or consists of the amino acid sequence having at least 90%, 91%, 92%, 93%, 94%, 95%, 96%, 97% or 98% sequence identity to SEQ ID NO: 4, the VL comprises or consists of the amino acid sequence having at least 90%, 91%, 92%, 93%, 94%, 95%, 96%, 97% or 98% sequence identity to SEQ ID NO: 5. In one aspect, the VH comprises or consists of the amino acid sequence having at least 90%, 91%, 92%, 93%, 94%, 95%, 96%, 97% or 98% sequence identity to SEQ ID NO: 12, the VL comprises or consists of the amino acid sequence having at least 90%, 91%, 92%, 93%, 94%, 95%, 96%, 97% or 98% sequence identity to SEQ ID NO: 13. In one aspect, the VH comprises or consists of the amino acid sequence having at least 90%, 91%, 92%, 93%, 94%, 95%, 96%, 97% or 98% sequence identity to SEQ ID NO: 20, the VL comprises or consists of the amino acid sequence having at least 90%, 91%, 92%, 93%, 94%, 95%, 96%, 97% or 98% sequence identity to SEQ ID NO: 21. In one aspect, the VH comprises or consists of the amino acid sequence of SEQ ID NO: 4; the VL comprises or consists of the amino acid sequence of SEQ ID NO: 5. In one aspect, the VH comprises or consists of the amino acid sequence of SEQ ID NO: 12; the VL comprises or consists of the amino acid sequence of SEQ ID NO: 13. In one aspect, the VH comprises or consists of the amino acid sequence of SEQ ID NO: 20; the VL comprises or consists of the amino acid sequence of SEQ ID NO: 21.

In one aspect, the VH and the VL are connected via a flexible polypeptide linker. In one aspect, the VH is fused by its N-terminus via a flexible polypeptide linker to the C-terminus of the VL (L-H). In one aspect, the VL is fused by its N-terminus via a flexible polypeptide linker to the C-terminus of the VH (H-L). In one aspect, the flexible polypeptide linker is (G₄S)ₙ (SEQ ID NO: 41), (SG₄)ₙ (SEQ ID NO: 42) or G₄(SG₄)ₙ (SEQ ID NO: 43), wherein "n" is 1, 2, 3, 4, 5, 6, 7 or 8, in particular 3. In one aspect, the flexible polypeptide linker comprises or consists of the amino acid sequence of SEQ ID NO: 28. In one aspect, the scFv comprises or consists of the amino acid sequence having at least 90%, 91%, 92%, 93%, 94%, 95%, 96%, 97%, 98% or 99% sequence identity to SEQ ID NO: 1, SEQ ID NO: 2 or SEQ ID NO: 3. In one aspect, the scFv comprises or consists of the amino acid sequence of SEQ ID NO: 1, SEQ ID NO: 2 or SEQ ID NO: 3. Table 2 provides the SEQ ID NOs: of exemplary scFvs.

**Table 2. SEQ ID NOs of the exemplary scFvs**

| No | HCDR1 | HCDR2 | HCDR3 | LCDR1 | LCDR2 | LCDR3 | VH | VL | Linker |
|---|---|---|---|---|---|---|---|---|---|
| Ofa-scFv | 6 | 7 | 8 | 9 | 10 | 11 | 4 | 5 | 28 |
| Obin-scFv | 14 | 15 | 16 | 17 | 18 | 19 | 12 | 13 | 28 |
| RTX-scFv | 22 | 23 | 24 | 25 | 26 | 27 | 20 | 21 | 28 |

### 2.2 Spacer

The spacer or hinge refers to the region that moves the extracellular antigen-binding domain away from the cell surface to enable cell contact, antigen binding and activation. The spacer in a CAR is located between the transmembrane domain and the extracellular antigen-binding domain. Exemplary hinge regions used in the CARs include the hinge region derived from the extracellular regions of type 1 membrane proteins such as CD8alpha, CD4, CD28 and CD7, which may be wild-type hinge regions from these proteins or may be modified. In one aspect, the spacer consists of the hinge domain from CD28. In one aspect, the spacer consists of the hinge domain (or stalk region) from wild-type CD28 . In one aspect, the spacer consists of an amino acid sequence having at least 90% sequence identity to SEQ ID NO: 30. In one aspect, the spacer consists of the amino acid sequence of SEQ ID NO: 30.

In one aspect, the spacer in the fusion protein includes at least a portion of an immunoglobulin constant region or variant or modified version thereof, such as a hinge region. In one aspect, the spacer includes at least a portion of IgG hinge. In one aspect, the spacer can be a chimeric polypeptide containing one or more hinge fragments derived from IgG1, IgG2, IgG3 and/or IgG4. In one aspect, the spacer can be a chimeric polypeptide containing one or more hinge fragments derived from IgG4. In one aspect, the spacer consists of an amino acid sequence that is 11, 12, 13, 14, 15, 16, 17, 18, 19 or 20 amino acid residues in length. In some embodiment, the spacer consists of an amino acid sequence that is 12-15 amino acid residues in length. In a preferred embodiment, the spacer consists of an amino acid sequence that is 12 amino acid residues in length. In one aspect, the spacer consists of an amino acid sequence that is at least about 90% identical to the amino acid sequence of SEQ ID NO: 31. In a particular embodiment, the spacer consists of the amino acid sequence of SEQ ID NO: 31.

### 2.3 Transmembrane domain

In one aspect, provided is the fusion protein as defined herein, wherein the transmembrane domain may be obtained from a naturally occurring protein or a synthetic, non-naturally occurring protein segment, e.g., a hydrophobic protein segment that is thermo-dynamically stable in a cell membrane. In one aspect, the protein segment is at least approximately 20 amino acids, e.g., at least 18, 19, 20, 21, 22, 23, 24, 25, 26, 27, 28, 29, 30, or more amino acids.

In one aspect, the transmembrane domain provided herein is derived from a Type I single-pass membrane protein. In one aspect, the transmembrane domains from multi-pass membrane proteins may also be compatible for use in the fusion proteins described herein. Multi-pass membrane proteins may comprise a complex (at least 2, 3, 4, 5, 6, 7 or more) alpha helices or a beta sheet structure. In one aspect, the N-terminus and the C-terminus of a multi-pass membrane protein are present on opposing sides of the lipid bilayer, e.g., the N-terminus of the protein is present on the cytoplasmic side of the lipid bilayer and the C-terminus of the protein is present on the extracellular side.

In one aspect, the transmembrane domain provided herein may comprise a transmembrane region and a cytoplasmic region located at the C-terminal side of the transmembrane domain. The cytoplasmic region of the transmembrane domain may comprise three or more amino acids and, in some embodiments, helps to orient the transmembrane domain in the lipid bilayer. In one aspect, one or more cysteine residues are present in the transmembrane region of the transmembrane domain. In one aspect, one or more cysteine residues are present in the cytoplasmic region of the transmembrane domain. In one aspect, the cytoplasmic region of the transmembrane domain comprises positively charged amino acids. In one aspect, the cytoplasmic region of the transmembrane domain comprises the amino acids arginine, serine, and lysine.

In one aspect, the transmembrane region of the transmembrane domain comprises hydrophobic amino acid residues. In one aspect, the transmembrane domain of the CAR provided herein comprises an artificial hydrophobic sequence. For example, a triplet of phenylalanine, tryptophan and valine may be present at the C terminus of the transmembrane domain. In one aspect, the transmembrane region comprises mostly hydrophobic amino acid residues, such as alanine, leucine, isoleucine, methionine, phenylalanine, tryptophan, or valine. In one aspect, the transmembrane region is hydrophobic. In some embodiments, the transmembrane region comprises a poly-leucine-alanine sequence.

In one aspect, the transmembrane domain comprises a transmembrane domain chosen from the transmembrane domain of an alpha, beta or zeta chain of a T-cell receptor, CD28, CD3 epsilon, CD45, CD4, CD5, CD8, CD9, CD16, CD22, CD33, CD37, CD64, CD80, CD86, CD134, CD137, CD154, KIRDS2, OX40, CD2, CD27, LFA-1 (CD11a, CD18), ICOS (CD278), GITR, CD40, BAFFR, HVEM (LIGHTR), SLAMF7, NKp80 (KLRFl), CD160, GPC3, IL-2R beta, IL-2R gamma, IL-7Ra, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4) , CD84, CD96 (Tactile), CEACAM1, CRT AM, Ly9 (CD229), CD160 (BY55) , PSGL1, CDIOO (SEMA4D), SLAMF6 (NTB-A, Lyl08), SLAM (SLAMF1, CD150, IPO-3), BLAME (SLAMF8), SELPLG (CD162), LTBR, PAG/Cbp, NKp44, NKp30, NKp46, NKG2D, and/or NKG2C. In one aspect, the transmembrane domain is derived from or comprises the transmembrane domain of CD8α, CD4, CD28, CD137, CD80, CD86, CD152 or PD1. In some specific embodiments, the transmembrane domain comprises the amino acid sequence having at least 88%, 92%, 96%, sequence identity to SEQ ID NO: 32. In a particular embodiment, the transmembrane domain comprises or consists of the amino acid sequence of SEQ ID NO: 32. In some specific embodiments, the transmembrane domain comprises the amino acid sequence having at least 88%, 92%, 96%, sequence identity to SEQ ID NO: 33. In a particular embodiment, the transmembrane domain comprises or consists of the amino acid sequence of SEQ ID NO: 33.

### 2.4 Intracellular signaling domain

In one aspect, provided is the fusion protein as defined herein, wherein the intracellular signaling domain is generally responsible for activation of at least one of the normal effector functions. In one aspect, the intracellular signaling domains include two distinct classes of cytoplasmic signaling sequences: those that initiate antigen-dependent primary activation through the TCR (primary intracellular signaling domains) and those that act in an antigen-independent manner to provide a secondary or co-stimulatory signal (secondary cytoplasmic domain, e.g., a co-stimulatory domain).

In one aspect, a primary signaling domain regulates primary activation of the TCR complex either in a stimulatory way, or in an inhibitory way. Primary intracellular signaling domains that act in a stimulatory manner may contain signaling motifs which are known as immunoreceptor tyrosine-based activation motifs (ITAMs). Examples of ITAM containing primary intracellular signaling domains that are of particular use in the invention include those of TCR zeta, FcR gamma, FcR beta, CD3 gamma, CD3 delta , CD3 epsilon, CD5, CD22, CD79a, CD79b, CD278 (also known as "ICOS"), FceRI, DAP10, DAP12, and CD66d. In one aspect, a CAR comprises an intracellular signaling domain, e.g., a primary signaling domain of CD3-zeta. In one aspect, a primary signaling domain comprises a modified ITAM domain, e.g., a mutated ITAM domain which has altered (e.g., increased or decreased) activity as compared to the native ITAM domain. In one aspect, a primary signaling domain comprises a modified ITAM-containing primary intracellular signaling domain, e.g., an optimized and/or truncated ITAM-containing primary intracellular signaling domain. In one aspect, the signaling domain of CD3-zeta is a mutant CD3zeta or a wild type human CD3zeta. In one aspect, a primary signaling domain comprises one, two, three, four or more ITAM motifs. In one aspect, the primary intracellular signaling domain is a functional mutant of the cytoplasmic signaling domain of CD3ζ containing one or more mutations, such as Q65K.

In one aspect, the intracellular signaling sequences within the cytoplasmic portion may be linked to each other in a random or specified order. In one aspect, the co-stimulatory molecule is a cell surface molecule other than an antigen receptor or its ligands that is required for an efficient response of lymphocytes to an antigen. Examples of such molecules include MHC class I molecule, TNF receptor proteins, Immunoglobulin-like proteins, cytokine receptors, signaling lymphocytic activation molecules (SEAM proteins), activating NK cell receptors, BTFA, a Toll ligand receptor, OX40, CD2, CD7, CD27, CD28, CD30, CD40, CDS, ICAM-l, FFA-l (CD11a/CD18), 4-1BB (CD137), B7-H3, CDS, ICAM-1, ICOS (CD278), GITR, BAFFR, FIGHT, HVEM, KIRDS2, SFAMF7, NKp80 (KFRF1), NKp44, NKp30, NKp46, CD19, CD4, CD8alpha, CD8beta, IF2Rbeta, IF2Rgamma, IF7R alpha, ITGA4, VFA1, CD49a, ITGA4, IA4, CD49D, ITGA6, VFA-6, CD49f, ITGAD, CD11d, ITGAE, CD103, ITGAF, FFA-1, ITGAM, CD11b, ITGAX, CD11c, ITGB1, CD29, ITGB2, CD18, FFA-1, ITGB7, NKG2D, NKG2C, TNFR2, TRANCE/RANKF, DNAM1 (CD226), SFAMF4 (CD244, 2B4), CD84, CD96 (Tactile), CEACAM1, CRTAM, Fy9 (CD229), CD160 (BY55), PSGF1, CD100 (SEMA4D), CD69, SFAMF6 (NTB-A, Fyl08), SEAM (SFAMF1, CD150, IPO-3), BFAME (SFAMF8), SEFPFG (CD162), FTBR, FAT, GADS, SFP-76, PAG/Cbp, CD19a, and a ligand that specifically binds with CD83, and the like.

In one aspect, the co-stimulatory signaling domains comprises up to 10 amino acid residue variations (e.g., 1, 2, 3, 4, 5, or 8) as compared to a wild-type counterpart. Such co-stimulatory signaling domains comprising one or more amino acid variations may be referred to as variants. Mutation of amino acid residues of the co-stimulatory signaling domain may result in an increase in signaling transduction and enhanced stimulation of immune responses relative to co-stimulatory signaling domains that do not comprise the mutation. Mutation of amino acid residues of the co-stimulatory signaling domain may result in a decrease in signaling transduction and reduced stimulation of immune responses relative to co-stimulatory signaling domains that do not comprise the mutation.

In one aspect, the one or more co-stimulatory signaling domains are selected from the group consisting of the signaling domains of CD27, CD28, CD137, OX40, CD30, CD40, CD3, lymphocyte function-associated antigen-1 (LFA-1), CD2, CD7, NKG2C, B7-H3 and ligands that specially bind to CD83. In one aspect, the intracellular signaling domain in the CAR of the present disclosure comprises a co-stimulatory signaling domain derived from CD137 (4-1BB). In one aspect, the intracellular signaling domain comprises a cytoplasmic signaling domain of CD3ζ and a co-stimulatory signaling domain of CD28 or CD137. In a particular embodiment, the intracellular signaling domain comprises a cytoplasmic signaling domain of CD3ζ and a co-stimulatory signaling domain of CD137.

### 2.5 CAR

In one aspect, provided is the fusion protein as defined herein, wherein the fusion protein is a CAR that comprises, from its N to C terminus in order: the extracellular antigen-binding domain, the spacer, the transmembrane domain and the intracellular signaling domain. In one aspect, the extracellular antigen-binding domain is an scFv that comprises, from its N to C terminus in order: a VH, the linker and a VL. In one aspect, the intracellular signaling domain comprises, from its N to C terminus in order: the signaling domain of CD137 and the cytoplasmic signaling domain of CD3ζ.

In one aspect, the extracellular antigen-binding domain is capable of specifically binding to the same epitope on CD20 as a reference antibody that comprises the amino acid sequence of SEQ ID NO: 1 and the spacer consists of a hinge domain from CD28. In one aspect, the extracellular antigen-binding domain comprises a VH comprising a HCDR1, a HCDR2 and a HCDR3 comprising the amino acid sequences of SEQ ID NO: 6, SEQ ID NO: 7 and SEQ ID NO: 8, respectively, and a VL comprising a LCDR1, a LCDR2 and a LCDR3 comprising the amino acid sequences of SEQ ID NO: 9, SEQ ID NO: 10 and SEQ ID NO: 11, respectively, and the spacer consists of the amino acid sequence of SEQ ID NO: 30. In a preferred embodiment, the extracellular antigen-binding domain is an scFv that comprises the amino acid sequence of SEQ ID NO: 1, and the spacer consists of the amino acid sequence of SEQ ID NO: 30.

In one aspect, the extracellular antigen-binding domain is capable of specifically binding to the same epitope on CD20 as a reference antibody that comprises the amino acid sequence of SEQ ID NO: 1 or SEQ ID NO: 2, and the spacer consists of a hinge domain from IgG4, wherein the spacer consisting of the hinge domain from IgG4 is at 12 amino acids in length. In one aspect, the extracellular antigen-binding domain comprises: a VH comprising a HCDR1, a HCDR2 and a HCDR3 comprising the amino acid sequences of SEQ ID NO: 6, SEQ ID NO: 7 and SEQ ID NO: 8, respectively, and a VL comprising a LCDR1, a LCDR2 and a LCDR3 comprising the amino acid sequences of SEQ ID NO: 9, SEQ ID NO: 10 and SEQ ID NO: 11, respectively; and the spacer consists of the amino acid sequence of SEQ ID NO: 31. In one aspect, the extracellular antigen-binding domain comprises a VH comprising a HCDR1, a HCDR2 and a HCDR3 comprising the amino acid sequences of SEQ ID NO: 14, SEQ ID NO: 15 and SEQ ID NO: 16, respectively, and a VL comprising a LCDR1, a LCDR2 and a LCDR3 comprising the amino acid sequences of SEQ ID NO: 17, SEQ ID NO: 18 and SEQ ID NO: 19, respectively; and the spacer consists of the amino acid sequence of SEQ ID NO: 31. In a preferred embodiment, the extracellular antigen-binding domain is an scFv that comprises the amino acid sequence of SEQ ID NO: 1 or SEQ ID NO: 2, and the spacer consists of the amino acid sequence of SEQ ID NO: 31.

In a particular embodiment, the CAR comprises the amino acid sequence of SEQ ID NO: 36, SEQ ID NO: 37 or SEQ ID NO: 38.

### III. Engineered Cells and preparation thereof

### 3.1 Cells

In one aspect, the present disclosure provides engineered cells comprising aforementioned fusion proteins, e.g., engineered immune cells. The source of the engineered immune cells of the present disclosure may be a patient to be treated (i.e., autologous cells) or from a donor who is not the patient to be treated (e.g., allogeneic cells).

In one aspect, the cell is an immunoresponsive cell. In one aspect, the cell is a cell of the lymphoid lineage. In one aspect, the engineered immune cells are engineered T cells. In one aspect, the T cells are derived from a mammalian subject. In one aspect, the T cells are derived from a primate subject, such as a human subject. Among the sub-types and subpopulations of T cells and/or of CD4+ and/or of CD8+ T cells are naive T (TN) cells, effector T cells (TEFF), memory T cells and sub-types thereof, such as stem cell memory T (TSCM), central memory T (TCM), effector memory T (TEM), or terminally differentiated effector memory T cells, tumor-infiltrating lymphocytes (TIL), immature T cells, mature T cells, helper T cells, cytotoxic T cells, mucosa-associated invariant T (MAIT) cells, naturally occurring and adaptive regulatory T (Treg) cells, helper T cells, such as TH1 cells, TH2 cells, TH3 cells, TH17 cells, TH9 cells, TH22 cells, follicular helper T cells, α/β T cells, and δ/γ T cells. Non-limiting examples of commercially available T-cell lines include lines BCL2 (AAA) Jurkat (ATCC^{®} CRL-2902^{™}), BCL2 (S70A) Jurkat (ATCC^{®} CRL-2900^{™}), BCL2 (S87A) Jurkat (ATCC^{®} CRL-2901^{™}), BCL2 Jurkat (ATCC^{®} CRL-2899^{™}), Neo Jurkat (ATCC^{®} CRL-2898^{™}), TALL-104 cytotoxic human T cell line (ATCC # CRL-11386). Further examples include but are not limited to mature T-cell lines, e.g., such as Deglis, EBT-8, HPB-MLp-W, HUT 78, HUT 102, Karpas 384, Ki 225, My-La, Se-Ax, SKW-3, SMZ-1 and T34; and immature T- cell lines, e.g., ALL-SIL, Be13, CCRF-CEM, CML-T1, DND-41, DU.528, EU-9, HD-Mar, HPB-ALL, H-SB2, HT-1, JK-T1, Jurkat, Karpas 45, KE-37, KOPT-K1, K-T1, L-KAW, Loucy, MAT, MOLT-1, MOLT 3, MOLT-4, MOLT 13, MOLT-16, MT-1, MT-ALL, P12/Ichikawa, Peer, PER0117, PER-255, PF-382, PFI-285, RPMI-8402, ST-4, SUP-T1 to T14, TALL-1, TALL-101, TALL-103/2, TALL-104, TALL-105, TALL-106, TALL-107, TALL-197, TK-6, TLBR-1, -2, -3, and -4, CCRF-HSB-2 (CCL-120.1), J.RT3-T3.5 (ATCC TIB-153), J45.01 (ATCC CRL-1990), J.CaM1.6 (ATCC CRL-2063), RS4;11 (ATCC CRL-1873), CCRF-CEM (ATCC CRM-CCL-119); and cutaneous T-cell lymphoma lines, e.g., HuT78 (ATCC CRM-TIB-161), MJ[G11] (ATCC CRL-8294), HuT102 (ATCC TIB-162). Non-limiting exemplary sources for such commercially available cell lines include the American Type Culture Collection (ATCC) (Manassas, VA), and the German Collection of Microorganisms and Cell Cultures.

In certain aspects, the cells are cytotoxic T cells (also known as TC, Cytotoxic T Lymphocyte, CTL, T Killer cell, a lytic T cell, CD8+ T cells or killer T cell). In certain aspects, the T cell is a CD4+T cell. In certain aspects, the T cell can be a CD4+T cell or a CD8+T cell. In certain aspects, the cell is a tumor-specific T cell.

In one aspect, the cells are natural killer (NK) cells, Natural Killer T (NKT) cells, cytokine-induced killer (CIK) cells, tumor-infiltrating lymphocytes (TILs), lymphokine- activated killer (LAK) cells, or the like. NK cells may either be isolated or obtained from a commercially available source. Non-limiting examples of commercial NK cell lines include lines NK-92 (ATCC^{®} CRL-2407^{™}), NK-92MI (ATCC^{®} CRL-2408^{™}). Further examples include but are not limited to NK lines HANK1, KHYG-1, NKL, NK-YS, NOI-90, and YT. Non-limiting exemplary sources for such commercially available cell lines include the American Type Culture Collection (ATCC) (Manassas, VA) and the German Collection of Microorganisms and Cell Cultures.

In one aspect, the cells are B cells, monocytes or granulocytes, e.g., myeloid cells, macrophages, neutrophils, dendritic cells, mast cells, eosinophils, and/or basophils.

In certain aspect, the cell is a cell of the myeloid lineage. Non-limiting examples of cells of the myeloid lineage include monocytes, macrophages, basophils, neutrophils, eosinophils, mast cell, erythrocytes, megakaryocytes, thrombocytes, and stem cells from which myeloid cells may be differentiated. In certain embodiments, the stem cell is a pluripotent stem cell (e.g., embryonic stem cell or induced pluripotent stem cell).

### 3.2. Methods for genetic engineering

In one aspect, the engineered cells are prepared by various methods of the transfer of polynucleotides encoding fusion proteins, e.g., antigen receptors, e.g., CARs. Physical methods include calcium phosphate precipitation, lipofection, particle bombardment, microinjection, electroporation, and the like. Biological methods include the use of DNA and RNA vectors, e.g., a viral vector, e.g., a lentiviral vector. Chemical methods include colloidal dispersion systems, such as macromolecule complexes, nanocapsules, microspheres, beads, and lipid-based systems including oil-in-water emulsions, micelles, mixed micelles, and liposomes. Exemplary methods are shown in Table 3. In some aspects, those well-known methods include transduction via viral e.g., retroviral or lentiviral, transposons, and electroporation.

**Table 3. Delivery methods for the genome editing systems**

| Delivery Mode | | Duration of Expression | Genome integration | Type of molecule delivered |
|---|---|---|---|---|
| Physical (e.g. electroporation) | | Transient | No | Nucleic acids and Proteins |
| Viral vector | retrovirus | Stable | Yes | RNA |
| | lentivirus | Stable | Yes | RNA |
| | adenovirus | Transient | No | DNA |
| | AAV | Stable | No | DNA |
| | Vaccinia Virus | Transient | No | DNA |
| | Herpes Simplex Virus | Stable | No | DNA |
| Non-viral | Cationic liposomes | Transient | | Nucleic acids and Proteins |
| | Polymeric nanoparticles | Transient | | Nucleic acids and Proteins |
| | Transposon | Stable | Yes | DNA |
| Biological non-viral delivery vehicles | Attenuated bacteria | Transient | No | Nucleic acids |

In one aspect, recombinant polynucleotides are transferred into cells using recombinant infectious virus particles, e.g., adenoviral vector, AAV vector, lentiviral vector, retroviral vector, such as gamma-retroviral vectors. In one aspect, the retroviral vector or lentiviral vector has a long terminal repeat sequence (LTR). In one aspect, the vectors are self-inactivating (SIN). In one aspect, the vectors are conditionally replicating (mobilizable) vectors. In one aspect, the lentiviral vectors are derived from human, feline or simian lentiviruses. In one aspect, the retroviral vectors are derived from murine retroviruses. In one aspect, the lentiviruses or retroviruses include those derived from any avian or mammalian cell source. In one aspect, the lentiviruses or retroviruses are amphotropic, meaning that they are capable of infecting host cells of several species, including humans. In one aspect, the gene to be expressed replaces the retroviral gag, pol and/or env sequences.

In one aspect, the vector comprising the polynucleotides encoding the fusion protein e.g., CAR, can contain a promoter and/or enhancer or regulatory elements to regulate expression of the encoded recombinant receptor. In one aspect, the promoter and/or enhancer or regulatory elements can be condition-dependent promoters, enhancers, and/or regulatory elements. In one aspect, the polynucleotides encoding the fusion protein can be operatively linked to a constitutive promoter. In one aspect, the promoter is selected from the group consisting of Cytomegalovirus (CMV) promoter, elongation factors-1 alpha (EF1α) promoter, ubiquitin C (UbiC) promoter, phosphor-glycerokinase (PGK) promoter, simian virus 40 early (SV40) promoter and chicken β-Actin promoter coupled with CMV early enhancer (CAGG).

In one aspect, the polynucleotides are operably linked to an inducible promoter. The inducible promoter can be induced by one or more conditions, such as a physical condition, microenvironment of the engineered immune effector cell, or the physiological state of the engineered immune effector cell, an inducer (i.e., an inducing agent) , or a combination thereof. In one aspect, the inducing condition does not induce the expression of endogenous genes in the engineered mammalian cell, and/or in the subject that receives the pharmaceutical composition. In one aspect, the inducing condition is selected from the group consisting of: inducer, irradiation (such as ionizing radiation, light), temperature (such as heat), redox state, tumor environment, and the activation state of the engineered mammalian cell.

In one aspect, the polynucleotides are operatively linked to a Woodchuck Hepatitis Virus (WHP) Posttranscriptional Regulatory Element (WPRE), located downstream of the polynucleotides.

In one aspect, the vector can contain a single promoter that drives the expression of one or more nucleic acid molecules. In one aspect, such nucleic acid molecules can be multi-cistronic. For example, in one aspect, transcription units can be engineered as a bi-cistronic unit containing an IRES (internal ribosome entry site), which allows co-expression of gene products (e.g., encoding a first and second CAR) by a message RNA from a single promoter. In one aspect, a single promoter may direct expression of an RNA that contains, in a single open reading frame (ORF), two or three genes (e.g. encoding a first and second CAR) separated from one another by sequences encoding a self-cleavage peptide or a protease recognition site. In one aspect, the self-cleavage peptide is selected from the group consisting of foot-and-mouth disease virus (F2A), equine rhinitis A virus (E2A), Thosea asigna virus (T2A) and porcine teschovirus-1 (P2A).

In one aspect, polynucleotides are transferred into T cells via electroporation. In one aspect, polynucleotides are transferred into T cells via transposition. In one aspect, polynucleotides are delivered by means of transposons including a Sleeping Beauty transposon system (SB) and/or a piggyBac (PB) transposon system.

Polynucleotides encoding fusion proteins may be associated with additional coding regions which encode secretory or signal peptides, which direct the secretion of the fusion protein. For example, if secretion of the fusion protein is desired, DNA encoding a signal sequence may be placed upstream of the fusion protein. Those of ordinary skill in the art are aware that polypeptides secreted by vertebrate cells generally have a signal peptide fused to the N-terminus of the polypeptide, which is cleaved from the translated polypeptide to produce a secreted or "mature" form of the polypeptide. In one aspect, the signal peptide comprises a sequence of a human CD2, CD3δ, CD3ε, CD3γ, CD3ζ, CD4, CD8α, CD19, CD28, CD37, CD45, 4-1BB, GM-CSFR, IL-2, CD33, Human IgKVIII, Human IgG2 H, Chymotrypsinogen, trypsinogen-2, HSA, Insulin or tPA signal peptide.

In one aspect, the polynucleotides encoding the fusion protein contain a nucleic acid sequence encoding one or more marker(s). In one aspect, the one or more marker(s) is a transduction marker, surrogate marker and/or a selection marker. In one aspect, the polynucleotides encoding the fusion protein contain a nucleic acid sequence encoding one or more additional fusion proteins that enhance and/or dampen the responses of the cells upon their adoptive transfer and encounter with ligands.

In one aspect, the polynucleotides can also encode one or more surrogate marker(s). In one aspect, the surrogate markers can include truncated forms of cell surface polypeptides, such as truncated forms that are non-functional and do not transduce or are not capable of transducing a signal or a signal ordinarily transduced by the full-length forms of the cell surface polypeptides, and/or do not internalize or are not capable of internalizing. In one aspect, the truncated cell surface polypeptides including truncated forms of growth factors or other receptors such as a truncated human epidermal growth factor receptor 2 (tHER2), a truncated epidermal growth factor receptor (EGFRt), a truncated prostate-specific membrane antigen (PSMA) or modified form thereof. EGFRt can be used to identify or select cells that have been engineered with the EGFRt fusion protein and an encoded exogenous protein, and/or to eliminate or separate cells expressing the encoded exogenous protein.

In one aspect, the marker is a fluorescent protein, such as green fluorescent protein (GFP), enhanced green fluorescent protein (EGFP), such as super-fold GFP (sfGFP), red fluorescent protein (RFP), such as tdTomato, mCherry, mStrawberry, AsRed2, DsRed or DsRed2, cyan fluorescent protein (CFP), blue green fluorescent protein (BFP), enhanced blue fluorescent protein (EBFP), and yellow fluorescent protein (YFP), and variants thereof, including species variants, monomeric variants, and codon-optimized and/or enhanced variants of the fluorescent proteins. In one aspect, the marker is or comprises an enzyme, such as a luciferase, the lacZ gene from E. coli, alkaline phosphatase, secreted embryonic alkaline phosphatase (SEAP), chloramphenicol acetyl transferase (CAT). Exemplary light-emitting reporter genes include luciferase (luc), β-galactosidase, chloramphenicol acetyltransferase (CAT), β-glucuronidase (GUS) or variants thereof.

In one aspect, the marker is a selection marker. In one aspect, the selection marker is a polypeptide that confers resistance to exogenous agents or drugs. In one aspect, the selection marker is an antibiotic resistance gene. In one aspect, the selection marker is an antibiotic resistance gene confers antibiotic resistance to a mammalian cell. In one aspect, the selection marker is selected from the group consisting of a Puromycin resistance gene, a Hygromycin resistance gene, a Blasticidin resistance gene, a Neomycin resistance gene, a Geneticin resistance gene or a Zeocin resistance gene or a modified form thereof.

Alternatively, various assays can be used to confirm the presence of the recombinant DNA sequence in the engineered cell, such as Southern and Northern blotting, RT-PCR and PCR. In one aspect, the fusion protein can be detected by the ability to recognize target cells, or the release of cytokines (e.g., interferon-γ, granulocyte/monocyte colony stimulating factor (GM-CSF), tumor necrosis factor α (TNF-α) or interleukin 2 (IL-2)). In addition, the function of fusion protein can be evaluated by measurement of cellular cytotoxicity.

### 3.3 Preparation of Engineered Cells

In one aspect, the present disclosure provides a manufacture process of the engineered cells. In one aspect, any known method for preparation may be used. In a particular aspect, the method includes transducing a population of isolated cells with the polynucleotide encoding the fusion protein and selecting a subpopulation of said isolated cells that have been successfully transduced with the polynucleotide thereby producing genetically modified cells, as described above.

In one aspect, the method includes acquisition, isolation, transduction, expansion steps. In a particular aspect, the method includes the following steps: (i) acquisition of an immune cell population (e.g. blood cells) (ii) isolation of a particular cell population (e.g. T cells and/or NK cells) (iii) transducing a population of isolated cells with the polynucleotide encoding the fusion protein; and (iv) expanding a subpopulation of said isolated cells that have been successfully transduced with said nucleic acid sequence of step (iii) thereby producing genetically modified cells. These different steps are more particularly described below.

### 3.3.1 Cell acquisition

In one aspect, the subject from which the cell is obtained for introduction of the fusion protein (e.g., CAR) is one having the disease or condition or in need of a cell therapy or to which cell therapy will be administered. In one aspect, the cells can be derived from a healthy donor.

In one aspect, the cells may be obtained from a sample, such as a biological sample. In one aspect, the samples are selected from whole blood, peripheral blood mononuclear cells (PBMCs), leukocytes, bone marrow, thymus, tissue biopsy, tumor tissue, leukemia tissue, lymphoma tissue, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. In one aspect, the cells are primary cells. In one aspect, cells from the circulating blood of a subject are obtained, e.g., by apheresis or leukapheresis. The resulting samples comprise lymphocytes, including T cells, monocytes, granulocytes, B cells, other nucleated white blood cells, red blood cells, and/or platelets, and in some aspects contain cells other than red blood cells and platelets.

### 3.3.2 Cell isolation

Various methods are readily available for isolating immune cells from a sample, for example using Life Technologies Dynabeads^{®} system; STEMcell Technologies EasySep^{™}, RoboSep^{™}, RosetteSep^{™}, SepMate^{™}; Miltenyi Biotec MACS^{™} cell separation kits, cell surface marker expression and other commercially available cell separation and isolation kits (e.g., ISOCELL from Pierce, Rockford, IL). Particular subpopulations of immune cells may be isolated through the use of beads or other binding agents available in such kits specific to unique cell surface markers. For example, MACS^{™} CD4+ and CD8+ MicroBeads may be used to isolate CD4+ and CD8+ T-cells.

In one aspect, isolation of the cells includes one or more non-affinity-based cell separation steps. In one aspect, cells are washed, centrifuged, and/or incubated in the presence of one or more reagents, for example, to remove unwanted components, enrich for desired components, lyse or remove cells sensitive to particular reagents. In one aspect, cells are separated based on one or more properties, such as density, adherent properties, size, sensitivity and/or resistance to particular components.

In one aspect, the blood cells collected from the subject are washed, e.g., to remove the plasma fraction and to place the cells in an appropriate buffer or media for subsequent processing steps. In one aspect, the cells are washed with phosphate buffered saline (PBS). In one aspect, the wash solution lacks calcium and may lack magnesium or may lack many if not all divalent cations. Initial activation steps in the absence of calcium may lead to magnified activation. In one aspect, a washing step is accomplished by a semi-automated "flow-through" centrifuge (for example, the Cobe 2991 cell processor, Baxter) according to the manufacturer's instructions. In one aspect, a washing step is accomplished by tangential flow filtration (TFF) according to the manufacturer's instructions. In one aspect, the cells are resuspended in a variety of biocompatible buffers after washing, such as, for example, Ca2+/Mg2+ free PBS. In certain embodiments, components of a blood cell sample are removed and the cells directly resuspended in culture media. In one aspect, the isolation includes density-based cell separation methods, such as the preparation of white blood cells from peripheral blood by lysing the red blood cells and centrifugation through a Percoll or Ficoll gradient.

In one aspect, the isolation methods include the separation of different cell types based on the expression or presence in the cell of one or more specific molecules, such as surface markers, e.g., surface proteins, intracellular markers, or nucleic acid. In one aspect, the separation is affinity- or immunoaffinity-based separation. The separation can be based on positive selection, in which the cells having bound the reagents are retained for further use, and/or negative selection, in which the cells having not bound to the antibody or binding partner are retained. The separation needs not result in 100% enrichment or removal of a particular cell population or cells expressing a particular marker.

In one aspect, one separation step can deplete cells expressing multiple markers simultaneously, such as by incubating cells with a plurality of antibodies or binding partners, each specific for a marker targeted for negative selection. Likewise, multiple cell types can simultaneously be positively selected by incubating cells with a plurality of antibodies or binding partners expressed on the various cell types. In one aspect, multiple rounds of separation steps are carried out, where the positively or negatively selected fraction from one step is subjected to another separation step, such as a subsequent positive or negative selection.

In some aspects, specific subpopulations of T cells, such as cells positive or expressing high levels of one or more surface markers, e.g., CD3+, CD28+, CD62L+, CCR7+, CD27+, CD127+, CD4+, CD8+, CD45RA+, and/or CD45RO+ T cells, are isolated by positive or negative selection techniques. In one aspect, T cells are isolated by incubation with anti-CD3/anti-CD28 conjugated particles or beads (e.g., DYNABEADS^{®} M-450 CD3/CD28 T Cell Expander, MACSiBeads^{™}, etc.). In one aspect, the time period of positive selection is about 30 minutes. In a further embodiment, the time period is at least 1, 2, 3, 4, 5, or 6 hours. In one aspect, the time period is 10 to 24 hours. In one aspect, the incubation time period is 24 hours. For isolation of a desired population of cells by positive or negative selection, the concentration of cells and particles can be varied. In certain embodiments, it may be desirable to significantly decrease the volume in which beads and cells are mixed together (i.e., increase the concentration of cells), to ensure maximum contact of cells and beads. In one aspect, greater than 100 million cells/mL is used.

In some aspects, T cells are separated from a sample by negative selection of markers expressed on non-T cells, such as B cells, monocytes, or other white blood cells, such as CD14. In some aspects, a CD4 or CD8 selection step is used to separate CD4+ helper and CD8+ cytotoxic T cells. Such CD4+ and CD8+ populations can be further sorted into sub-populations by positive or negative selection for markers expressed or expressed to a relatively higher degree on one or more naive, memory, and/or effector T cell subpopulations.

In some aspects, CD8+ cells are further enriched for or depleted of naive, central memory, effector memory, and/or central memory stem cells, such as by positive or negative selection based on surface antigens associated with the respective subpopulation. In some aspects, enrichment for central memory T (TCM) cells is carried out to increase efficacy, such as to improve long-term survival, expansion, and/or engraftment following administration. In some aspects, combining TCM-enriched CD8+ T cells and CD4+ T cells further enhances efficacy.

In some aspects, memory T cells are present in both CD62L+ and CD62L- subsets of CD8+ peripheral blood lymphocytes. PBMC can be enriched for or depleted of CD62L- CD8+ and/or CD62L+CD8+ fractions, such as using anti-CD8 and anti-CD62L antibodies.

In some aspects, the enrichment for central memory T (TCM) cells is based on positive or high surface expression of CD45RO, CD62L, CCR7, CD28, CD3, and/or CD127. In some aspects, it is based on negative selection for cells expressing or highly expressing CD45RA and/or granzyme B. In some aspects, isolation of a CD8+ population enriched for TCM cells is carried out by depletion of cells expressing CD4, CD14, CD45RA, and positive selection or enrichment for cells expressing CD62L. In one aspect, enrichment for central memory T (TCM) cells is carried out starting with a negative fraction of cells selected based on CD4 expression, which is subjected to a negative selection based on expression of CD14 and CD45RA, and a positive selection based on CD62L.

In one aspect, the enrichment for NK cells is based on positive or high surface expression of CD56 and CD16 and on the negative expression of CD3 and/or optionally on the presence of NKp46 or NKp30 receptors.

In one aspect, the sample or composition of cells to be separated is incubated with small, magnetizable or magnetically responsive material, such as magnetically responsive particles or microparticles, such as paramagnetic beads (e.g., Dynabeads^{®} or MACS^{®} beads). The magnetically responsive material, e.g., particle, generally is directly or indirectly attached to a binding partner, e.g., an antibody, that specifically binds to a molecule, e.g., surface marker, present on the cell, cells, or population of cells that it is desired to separate, e.g., that it is desired to negatively or positively select. In one aspect, the sample is placed in a magnetic field, and those cells having magnetically responsive or magnetizable particles attached thereto will be attracted to the magnet and separated from the unlabeled cells. For positive selection, cells that are attracted to the magnet are retained; for negative selection, cells that are not attracted (unlabeled cells) are retained. In some aspects, a combination of positive and negative selection is performed during the same selection step, where the positive and negative fractions are retained and further processed or subject to further separation steps.

In one aspect, the magnetically responsive particles are left attached to the cells that are to be subsequently incubated, cultured and/or engineered; in some aspects, the particles are left attached to the cells for administration to a patient. In one aspect, the magnetically responsive particles are removed from the cells. Methods for removing magnetically responsive particles from cells are known and include, e.g., the use of competing non-labeled antibodies, magnetically responsive particles or antibodies conjugated to cleavable linkers, etc. In one aspect, the magnetically responsive particles are biodegradable.

In one aspect, the affinity-based selection is via magnetic-activated cell sorting (MACS^{®}) (Miltenyi Biotec, Auburn, CA). Magnetic Activated Cell Sorting (MACS^{®}) systems are capable of high-purity selection of cells having magnetized particles attached thereto. In certain embodiments, MACS^{®} operates in a mode wherein the non-target and target species are sequentially eluted after the application of the external magnetic field. That is, the cells attached to magnetized particles are held in place while the unattached species are eluted. Then, after this first elution step is completed, the species that were trapped in the magnetic field and were prevented from being eluted are freed in some manner such that they can be eluted and recovered. In certain embodiments, the non-target cells are labelled and depleted from the heterogeneous population of cells.

In one aspect, a cell population described herein is collected and enriched (or depleted) via flow cytometry, in which cells stained for multiple cell surface markers are carried in a fluidic stream. In one aspect, a cell population described herein is collected and enriched (or depleted) via preparative scale (FACS)-sorting. In certain embodiments, a cell population described herein is collected and enriched (or depleted) by use of microelectromechanical systems (MEMS) chips in combination with a FACS-based detection system. In both cases, cells can be labeled with multiple markers, allowing for the isolation of well-defined T cell subsets at high purity.

In one aspect, the preparation methods include steps for freezing, e.g., cryopreserving, the cells, either before or after isolation, incubation, and/or engineering. In one aspect, the freeze and subsequent thaw step removes granulocytes and, to some extent, monocytes in the cell population. In one aspect, the cells are suspended in a freezing solution, e.g., following a washing step to remove plasma and platelets. Any of a variety of known freezing solutions and parameters in some aspects may be used. One example involves using PBS containing 20% DMSO and 8% human serum albumin (HSA), or other suitable cell freezing media. This is then diluted 1:1 with media so that the final concentration of DMSO and HSA are 10% and 4%, respectively. The cells are then frozen to -80 °C at a rate of 1 °C per minute and stored in the vapor phase of a liquid nitrogen storage tank. In one aspect, cryopreserved cells are thawed and washed as described herein and allowed to rest for one hour at room temperature prior to activation.

### 3.3.3 Cell expansion

In one aspect, the provided methods include cultivation, incubation, culture, and/or genetic engineering steps. In one aspect, the cells are incubated and/or cultured prior to or in connection with genetic engineering. The incubation steps can include culture, cultivation, stimulation, activation, and/or propagation. In one aspect, the cells are incubated in the presence of stimulating conditions or a stimulatory agent. The conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In one aspect, the stimulating conditions or agents include one or more agent, e.g., ligand, which is capable of stimulating or activating an intracellular signaling domain of a TCR complex. In some aspects, the agent turns on or initiates TCR/CD3 intracellular signaling cascade in a T cell. Such agents can include antibodies, such as those specific for a TCR component and/or co-stimulatory receptor, e.g., anti-CD3, anti-CD28, for example, bound to solid support such as a bead (e.g., Dynabeads^{®}), and/or one or more cytokines. In one aspect, a concentration of cells of 10, 15, 20, 25, 30, 35, 40, 45, 50, 75, 80, 85, 90, 95, 100 125 or 150 million cells/mL is used.

In one aspect, the mixture may be cultured for several hours (about 3 hours) to about 14 days or any hourly integer value in between. In another embodiment, the mixture may be cultured for 21 days. In one embodiment, the beads and the T cells are cultured together for about eight days. In another embodiment, the beads and cells are cultured together for 2-3 days. Several cycles of stimulation may also be desired such that culture time of T cells can be 60 days or more. Conditions appropriate for T cell culture include an appropriate media that may contain factors necessary for proliferation and viability, including interleukin-2 (IL-2), insulin, IFN-γ, IL-4, IL-7, GM-CSF, IL-10, IL-12, IL-15, TGFβ, and TNF-α or any other additives. Other additives for the growth of cells include, but are not limited to, surfactant, plasmanate, and reducing agents such as N-acetylcysteine and 2-mercaptoethanol. Media can include RPMI 1640, AIM-V, DMEM, MEM, α-MEM, F-12, X-Vivo 15, and X-Vivo 20, optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of T cells. Antibiotics, e.g., penicillin and streptomycin, are included only in experimental cultures, not in cultures of cells that are to be infused into a subject. The target cells are maintained under conditions necessary to support growth, for example, an appropriate temperature (e.g., 37 °C) and atmosphere (e.g., air plus 5% CO₂).

In one aspect, NK cell populations can be expanded in vitro using interleukin-2 (IL-2) IL-15, IL-15/IL-15RA complex, IL-18 and IL-12. In one aspect, the NK cells are *ex vivo* expanded for at least about 5 days, for example, not less than about 10 days, not less than about 15 days, or not less than about 20 days before administration to the patient.

### IV. PHARMACEUTICAL COMPOSITION

Pharmaceutical compositions of the present disclosure may comprise engineered cells in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present disclosure are in one aspect formulated for intravenous administration.

In one aspect, the pharmaceutical composition is substantially free of contaminants, e.g., there are no detectable levels of contaminants, e.g., those selected from the group consisting of endotoxin, mycoplasma, replication competent lentivirus (RCL), p24, VSV-G nucleic acid, HIV gag, residual anti-CD3/anti- CD28 coated beads, mouse antibodies, pooled human serum, bovine serum albumin, bovine serum, culture media components, vector packaging cell or plasmid components, a bacterium and a fungus. In one aspect, the bacterium is at least one selected from the group consisting of Alcaligenes faecalis, Candida albicans, Escherichia coli, Haemophilus influenza, Neisseria meningitides, Pseudomonas aeruginosa, Staphylococcus aureus, Streptococcus pneumonia, and Streptococcus pyogenes group A.

When "an immunologically effective amount", "an anti-tumor effective amount", "a tumor- inhibiting effective amount", or "therapeutic amount" is indicated, the precise amount of the compositions of the present disclosure to be administered can be determined by a physician with consideration of individual differences in age, weight, tumor size, extent of infection or metastasis, and condition of the patient (subject). It can generally be stated that a pharmaceutical composition comprising the T cells described herein may be administered at a dosage of 10⁴ to 10⁹ cells/kg body weight, in some instances 10⁵ to 10⁶ cells/kg body weight, including all integer values within those ranges. T cell compositions may also be administered one time or multiple times at these dosages. The cells can be administered by using infusion techniques that are commonly known in immunotherapy.

### V. METHOD OF TREATMENT

Engineered cells or pharmaceutical compositions of the present disclosure may be administered in a manner appropriate to the disease to be treated (or prevented). The diseases may comprise solid tumors, such as sarcomas and carcinomas, include fibrosarcoma, myxosarcoma, liposarcoma, chondrosarcoma, osteosarcoma, and other sarcomas, synovioma, mesothelioma, Ewing's tumor, leiomyosarcoma, rhabdomyosarcoma, colon carcinoma, lymphoid malignancy, pancreatic cancer, breast cancer, lung cancers, ovarian cancer, prostate cancer, hepatocellular carcinoma, squamous cell carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, melanoma, and CNS tumors (such as a glioma (such as brainstem glioma and mixed gliomas), glioblastoma (also known as glioblastoma multiforme) astrocytoma, CNS lymphoma, germinoma, medulloblastoma, Schwannoma craniopharyogioma, ependymoma, pinealoma, hemangioblastoma, acoustic neuroma, oligodendroglioma, menangioma, neuroblastoma, retinoblastoma and brain metastases); the non-solid tumors, such as leukemias, including acute leukemias (such as acute lymphocytic leukemia, acute myelocytic leukemia, acute myelogenous leukemia and myeloblastic, promyelocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

In one aspect, the subject has a relapsed or refractory disease or disorder associated with CD20. In one aspect, the subject has been previously treated with at least one regimen comprising a medicament directing against CD20. In one aspect, the subject has been relapsed or refractory to the treatment comprising an anti-CD20 monoclonal antibody. In some embodiments, the anti-CD20 monoclonal antibody is Ofatumumab, Obinutuzumab or Rituximab. In one aspect, the subject has disease progression within 6, 12, 18, 24, 30 or 36 months after initiation of prior therapy. In one aspect, the subject has not been treated with the anti-CD20 monoclonal antibody for at least 4 weeks (e.g., 4 weeks, 6 weeks, 8 weeks, 10 weeks, 12 weeks, 24 weeks, 36 weeks, 48 weeks, 60 weeks, or more) prior to being administered the effective amount of engineered cells or pharmaceutical compositions of this disclosure.

The disease may also comprises autoimmune disease selected from the group consisting of inflammatory responses such as inflammatory skin diseases including psoriasis and dermatitis (e.g. atopic dermatitis); systemic scleroderma and sclerosis; responses associated with inflammatory bowel disease (such as Crohn's disease and ulcerative colitis); respiratory distress syndrome (including adult respiratory distress syndrome; ARDS); meningitis; encephalitis; uveitis; colitis; glomerulonephritis; allergic conditions such as eczema and asthma and other conditions involving infiltration of T-cells and chronic inflammatory responses; atherosclerosis; leukocyte adhesion deficiency; rheumatoid arthritis; systemic lupus erythematosus (SLE); Wegener's disease; diabetes mellitus (e.g. Type I diabetes mellitus or insulin dependent diabetes mellitus); multiple sclerosis; Reynaud's syndrome; autoimmune thyroiditis; allergic encephalomyelitis; Sjogren's syndrome; juvenile onset diabetes; and immune responses associated with acute and delayed hypersensitivity mediated by cytokines and T-lymphocytes typically found in tuberculosis, sarcoidosis, polymyositis, granulomatosis and vasculitis; pernicious anemia (Addison's disease); diseases involving leukocyte diapedesis; central nervous system (CNS) inflammatory disorder; multiple organ injury syndrome; hemolytic anemia; myasthenia gravis; antigen-antibody complex mediated diseases; anti-glomerular basement membrane disease; anti-phospholipid syndrome; allergic neuritis; Graves' disease; Lambert-Eaton myasthenic syndrome; pemphigoid bullous; pemphigus; autoimmune polyendocrinopathies; Reiter's disease; stiff-man syndrome; Bechet disease; giant cell arteritis; immune complex nephritis; IgA nephropathy; IgM polyneuropathies; immune thrombocytopenic purpura (ITP) or autoimmune thrombocytopenia. In one embodiment the medicament is for treatment of multiple sclerosis. In one preferred embodiment the medicament is for the treatment of secondary progressive multiple sclerosis.

The quantity and frequency of administration will be determined by the condition of the patient, and the type and severity of the patient's disease, although appropriate dosages may be determined by clinical trials. The administration of the compositions may be carried out in any convenient manner, including by aerosol injection, ingestion, transfusion, implantation or transplantation. The compositions described herein may be administered to a patient transarterially, subcutaneously, intradermally, intratumorally, intranodally, intramedullary, intramuscularly, by intravenous (i.v.) injection, or intraperitoneally. In one aspect, the T cell compositions of the present invention are administered to a patient by intradermal or subcutaneous injection. In one aspect, the compositions are directly injected into an organ of interest (e.g., an organ affected by a neoplasm). Alternatively, the compositions are provided indirectly to the organ of interest, for example, by administration into the circulatory system (e.g., the tumor vasculature). Expansion and differentiation agents can be provided prior to, during or after administration of the cells or compositions to increase production of T cells or NK cells in vitro or in vivo.

In one aspect, lymphodepletion is performed on a subject, e.g., prior to administering one or more cells described herein. In one aspect, the lymphodepletion comprises administering one or more of melphalan, cytoxan, cyclophosphamide, and fludarabine.

In one aspect, the engineered cells are administered as part of a combination treatment, such as simultaneously with or sequentially with, in any order, another therapeutic intervention, such as an antibody or engineered cell or receptor or agent, such as a cytotoxic or therapeutic agent. The cells in some embodiments are co-administered with one or more additional therapeutic agents or in connection with another therapeutic intervention, either simultaneously or sequentially in any order. In one aspect, the cells are co-administered with another therapy sufficiently close in time such that the cell populations enhance the effect of one or more additional therapeutic agents, or vice versa. In one aspect, the cells are administered prior to the one or more additional therapeutic agents. In one aspect, the cells are administered after to the one or more additional therapeutic agents, such as anti-cancer agents. In the context of the present disclosure, it is contemplated that cell therapy could be used similarly in conjunction with chemotherapeutic, radiotherapeutic, or immunotherapeutic intervention, as well as pro-apoptotic or cell cycle regulating agents such as immune checkpoint inhibitor.

Alternatively, the present therapy may precede or follow the other agent treatment by intervals ranging from minutes to weeks. In embodiments where the other agent and the cell of present disclosure are applied separately to the individual, one would generally ensure that a significant period of time did not expire between the times of each delivery, such that the agent and therapy would still be able to exert an advantageously combined effect on the cell. In such instances, it is contemplated that one may contact the cell with both modalities within about 12-24 h of each other and, more preferably, within about 6-12 h of each other. In some situations, it may be desirable to extend the time period for treatment significantly, however, where several days (2, 3, 4, 5, 6 or 7) to several week (1, 2, 3, 4, 5, 6, 7 or 8) lapse between the respective administrations. It is expected that the treatment cycles would be repeated if necessary. It also is contemplated that various standard therapies, as well as surgical intervention, may be applied in combination with the cell therapy.

### VI. Sequences

**Table 4. Sequences of the present disclosure**

| SEQ ID No | Name | Sequence |
|---|---|---|
| 1 | Ofa-scFv | EVQLVESGGGLVQPGRSLRLSCAASGFTFNDYAMHWVRQAPGKGLE WVSTISWNSGSIGYADSVKGRFTISRDNAKKSLYLQMNSLRAEDTAL YYCAKDIQYGNYYYGMDVWGQGTTVTVSSGGGGSGGGGSGGGGSE IVLTQSPATLSLSPGERATLSCRASQSVSSYLAWYQQKPGQAPRLLIY DASNRATGIPARFSGSGSGTDFTLTISSLEPEDFAVYYCQQRSNWPITF GQGTRLEIKRTV |
| 2 | Obin-scFv | QVQLVQSGAEVKKPGSSVKVSCKASGYAFSYSWINWVRQAPGQGLE WMGRIFPGDGDTDYNGKFKGRVTITADKSTSTAYMELSSLRSEDTAV YYCARNVFDGYWLVYWGQGTLVTVSSGGGGSGGGGSGGGGSDIVM TQTPLSLPVTPGEPASISCRSSKSLLHSNGITYLYWYLQKPGQSPQLLIY QMSNLVSGVPDRFSGSGSGTDFTLKISRVEAEDVGVYYCAQNLELPY TFGGGTKVEIKRTV |
| 3 | RTX-scFv | QVQLQQPGAELVKPGASVKMSCKASGYTFTSYNMHWVKQTPGRGL EWIGAIYPGNGDTSYNQKFKGKATLTADKSSSTAYMQLSSLTSEDSA VYYCARSTYYGGDWYFNVWGAGTTVTVSGGGGSGGGGSGGGGSQI VLSQSPAILSASPGEKVTMTCRASSSVSYIHWFQQKPGSSPKPWIYATS NLASGVPVRFSGSGSGTSYSLTISRVEAEDAATYYCQQWTSNPPTFGG GTKLEIKR |
| 4 | Ofa-VH | EVQLVESGGGLVQPGRSLRLSCAASGFTFNDYAMHWVRQAPGKGLE WVSTISWNSGSIGYADSVKGRFTISRDNAKKSLYLQMNSLRAEDTAL YYCAKDIQYGNYYYGMDVWGQGTTVTVSS |
| 5 | Ofa-VL | EIVLTQSPATLSLSPGERATLSCRASQSVSSYLAWYQQKPGQAPRLLIY DASNRATGIPARFSGSGSGTDFTLTISSLEPEDFAVYYCQQRSNWPITF GQGTRLEIKRTV |
| 6 | Ofa HCDR1 | DYAMH |
| 7 | Ofa HCDR2 | TISWNSGSIGYADSVKG |
| 8 | Ofa HCDR3 | DIQYGNYYYGMDV |
| 9 | Ofa LCDR1 | RASQSVSSYLA |
| 10 | Ofa LCDR2 | DASNRAT |
| 11 | Ofa LCDR3 | QQRSNWPIT |
| 12 | Obin-VH | QVQLVQSGAEVKKPGSSVKVSCKASGYAFSYSWINWVRQAPGQGLE WMGRIFPGDGDTDYNGKFKGRVTITADKSTSTAYMELSSLRSEDTAV YYCARNVFDGYWLVYWGQGTLVTVSS |
| 13 | Obin-VL | DIVMTQTPLSLPVTPGEPASISCRSSKSLLHSNGITYLYWYLQKPGQSP QLLIYQMSNLVSGVPDRFSGSGSGTDFTLKISRVEAEDVGVYYCAQN LELPYTFGGGTKVEIKRTV |
| 14 | Obin HCDR1 | YSWIN |
| 15 | Obin HCDR2 | RIFPGDGDTDYNGKFKG |
| 16 | Obin HCDR3 | NVFDGYWLVY |
| 17 | Obin LCDR1 | RSSKSLLHSNGITYLY |
| 18 | Obin LCDR2 | QMSNLVS |
| 19 | Obin LCDR3 | AQNLELPYT |
| 20 | RTX-VH | QVQLQQPGAELVKPGASVKMSCKASGYTFTSYNMHWVKQTPGRGL EWIGAIYPGNGDTSYNQKFKGKATLTADKSSSTAYMQLSSLTSEDSA VYYCARSTYYGGDWYFNVWGAGTTVTVS |
| 21 | RTX-VL | QIVLSQSPAILSASPGEKVTMTCRASSSVSYIHWFQQKPGSSPKPWIYA TSNLASGVPVRFSGSGSGTSYSLTISRVEAEDAATYYCQQWTSNPPTF GGGTKLEIKR |
| 22 | RTX HCDR1 | SYNMH |
| 23 | RTX HCDR2 | AIYPGNGDTSYNQKFKG |
| 24 | RTX HCDR3 | STYYGGDWYFNV |
| 25 | RTX LCDR1 | RASSSVSYIH |
| 26 | RTX LCDR2 | ATSNLAS |
| 27 | RTX LCDR3 | QQWTSNPPT |
| 28 | linker | GGGGSGGGGSGGGGS |
| 29 | CD8 hinge | PPTPAPTIASQPLSLRPEACRPAAGGAVHTRGLDFACD |
| 30 | CD28 hinge | KIEVMYPPPYLDNEKSNGTIIHVKGKHLCPSPLFPGPSKP |
| 31 | IgG4 hinge | ESKYGPPCPPCP |
| 32 | CD8 TM | IYIWAPLAGTCGVLLLSLVITLYC |
| 33 | CD28 TM | MFWVLVVVGGVLACYSLLVTVAFIIFWV |
| 34 | 4-1BB | KRGRKKLLYIFKQPFMRPVQTTQEEDGCSCRFPEEEEGGCEL |
| 35 | CD3ζ | RVKFSRSADAPAYQQGQNQLYNELNLGRREEYDVLDKRRGRDPEMG GKPRRKNPQEGLYNELQKDKMAEAYSEIGMKGERRRGKGHDGLYQ GLSTATKDTYDALHMQALPPR |
| 36 | CAR-8 | EVQLVESGGGLVQPGRSLRLSCAASGFTFNDYAMHWVRQAPGKGLE WVSTISWNSGSIGYADSVKGRFTISRDNAKKSLYLQMNSLRAEDTAL YYCAKDIQYGNYYYGMDVWGQGTTVTVSSGGGGSGGGGSGGGGSE IVLTQSPATLSLSPGERATLSCRASQSVSSYLAWYQQKPGQAPRLLIY DASNRATGIPARFSGSGSGTDFTLTISSLEPEDFAVYYCQQRSNWPITF GQGTRLEIKRTVKIEVMYPPPYLDNEKSNGTIIHVKGKHLCPSPLFPGP SKPMFWVLVVVGGVLACYSLLVTVAFIIFWVKRGRKKLLYIFKQPFM |
| | | RPVQTTQEEDGCSCRFPEEEEGGCELRVKFSRSADAPAYQQGQNQLY NELNLGRREEYDVLDKRRGRDPEMGGKPRRKNPQEGLYNELQKDK MAEAYSEIGMKGERRRGKGHDGLYQGLSTATKDTYDALHMQALPPR |
| 37 | CAR-4 | QVQLVQSGAEVKKPGSSVKVSCKASGYAFSYSWINWVRQAPGQGLE WMGRIFPGDGDTDYNGKFKGRVTITADKSTSTAYMELSSLRSEDTAV YYCARNVFDGYWLVYWGQGTLVTVSSGGGGSGGGGSGGGGSDIVM TQTPLSLPVTPGEPASISCRSSKSLLHSNGITYLYWYLQKPGQSPQLLIY QMSNLVSGVPDRFSGSGSGTDFTLKISRVEAEDVGVYYCAQNLELPY TFGGGTKVEIKRTVESKYGPPCPPCPMFWVLVVVGGVLACYSLLVTV AFIIFWVKRGRKKLLYIFKQPFMRPVQTTQEEDGCSCRFPEEEEGGCE LRVKFSRSADAPAYQQGQNQLYNELNLGRREEYDVLDKRRGRDPEM GGKPRRKNPQEGLYNELQKDKMAEAYSEIGMKGERRRGKGHDGLY QGLSTATKDTYDALHMQALPPR |
| 38 | CAR-7 | EVQLVESGGGLVQPGRSLRLSCAASGFTFNDYAMHWVRQAPGKGLE WVSTISWNSGSIGYADSVKGRFTISRDNAKKSLYLQMNSLRAEDTAL YYCAKDIQYGNYYYGMDVWGQGTTVTVSSGGGGSGGGGSGGGGSE IVLTQSPATLSLSPGERATLSCRASQSVSSYLAWYQQKPGQAPRLLIY DASNRATGIPARFSGSGSGTDFTLTISSLEPEDFAVYYCQQRSNWPITF GQGTRLEIKRTVESKYGPPCPPCPMFWVLVVVGGVLACYSLLVTVAF IIFWVKRGRKKLLYIFKQPFMRPVQTTQEEDGCSCRFPEEEEGGCELR VKFSRSADAPAYQQGQNQLYNELNLGRREEYDVLDKRRGRDPEMGG KPRRKNPQEGLYNELQKDKMAEAYSEIGMKGERRRGKGHDGLYQG LSTATKDTYDALHMQALPPR |
| 39 | Signal peptide | METDTLLLWVLLLWVPGSTG |
| 40 | EGFRt | MLLLVTSLLLCELPHPAFLLIPRKVCNGIGIGEFKDSLSINATNIKHFKN CTSISGDLHILPVAFRGDSFTHTPPLDPQELDILKTVKEITGFLLIQAWP ENRTDLHAFENLEIIRGRTKQHGQFSLAVVSLNITSLGLRSLKEISDGD VIISGNKNLCYANTINWKKLFGTSGQKTKIISNRGENSCKATGQVCHA LCSPEGCWGPEPRDCVSCRNVSRGRECVDKCNLLEGEPREFVENSECI QCHPECLPQAMNITCTGRGPDNCIQCAHYIDGPHCVKTCPAGVMGEN NTLVWKYADAGHVCHLCHPNCTYGCTGPGLEGCPTNGPKIPSIATGM VGALLLLLVVALGIGLFM |
| 41 | linker-3 | (G₄S)ₙ, wherein "n" is 1, 2, 3, 4, 5, 6, 7 or 8, in particular 3. |
| 42 | linker-4 | (SG₄)ₙ, wherein "n" is 1, 2, 3, 4, 5, 6, 7 or 8, in particular 3. |
| 43 | linker-5 | G₄(SG₄)ₙ, wherein "n" is 1, 2, 3, 4, 5, 6, 7 or 8, in particular 3. |

### VII. Example

The following examples are included for illustrative purposes only and are not intended to limit the scope of the invention.

### Recombinant DNA techniques

Standard methods were used to manipulate DNA as described in Sambrook et al, Molecular cloning: A laboratory manual; Cold Spring Harbor Laboratory Press, Cold Spring Harbor, New York, 1989. The molecular biological reagents were used according to the manufacturer's instructions. General information regarding the nucleotide sequences of human immunoglobulin light and heavy chains is given in: Kabat, E.A. et al, (1991) Sequences of Proteins of Immunological Interest, Fifth Ed., NIH Publication No 91-3242.

### DNA sequencing

DNA sequences were determined by double strand sequencing.

### Gene synthesis

Desired gene segments, where required, were either generated by PCR using appropriate templates or were synthesized from synthetic oligonucleotides and PCR products by automated gene synthesis. The gene segments flanked by unique restriction endonuclease cleavage sites were cloned into standard cloning/sequencing vectors. The plasmid DNA was purified from transformed bacteria and concentration determined by UV spectroscopy. The DNA sequence of the subcloned gene fragments was confirmed by DNA sequencing. Gene segments were designed with suitable restriction sites to allow subcloning into the respective expression vectors. All constructs were designed with a 5'-end DNA sequence coding for a leader peptide which targets proteins for secretion in eukaryotic cells.

### Example 1: Generation and assessment of CD20 CARs in Jurkat cells

CARs in Table 5 with signal peptides were formed through the fusion of a signal peptide to either RTX-scFv, Obin-scFv, Ofa-scFv followed by three different spacers and a transmembrane derived from human CD28 or CD8 and an endodomain encoding the signaling motifs from 41BB and CD3ζ. Each CAR was co-expressed with a truncated EGFR (EGFRt, SEQ ID NO: 40), used as a transduction marker. Both CAR and EGFRt were encoded from a single promoter construct separated by a self-cleaving T2A peptide. The signal peptides fused to the N-terminus of CARs were shown in SEQ ID NO: 39.

**Table 5. Structures of candidate CARs (from N- to C-terminus)**

| No | Antigen-binding domain | Spacer | Transmembrane domain | Co-stimulatory domain | Primary intracellular signaling domain |
|---|---|---|---|---|---|
| CAR-1 | RTX-scFv | IgG4 hinge | CD28 TM | | |
| CAR-2 | RTX-scFv | CD28 hinge | CD28 TM | | |
| CAR-3 | RTX-scFv | CD8 hinge | CD8 TM | | |
| CAR-4 | Obin-scFv | IgG4 hinge | CD28 TM | | |
| CAR-5 | Obin-scFv | CD28 hinge | CD28 TM | 4-1BB | CD3ζ |
| CAR-6 | Obin-scFv | CD8 hinge | CD8 TM | | |
| CAR-7 | Ofa-scFv | lgG4 hinge | CD28 TM | | |
| CAR-8 | Ofa-scFv | CD28 hinge | CD28 TM | | |
| CAR-9 | Ofa-scFv | CD8 hinge | CD8 TM | | |

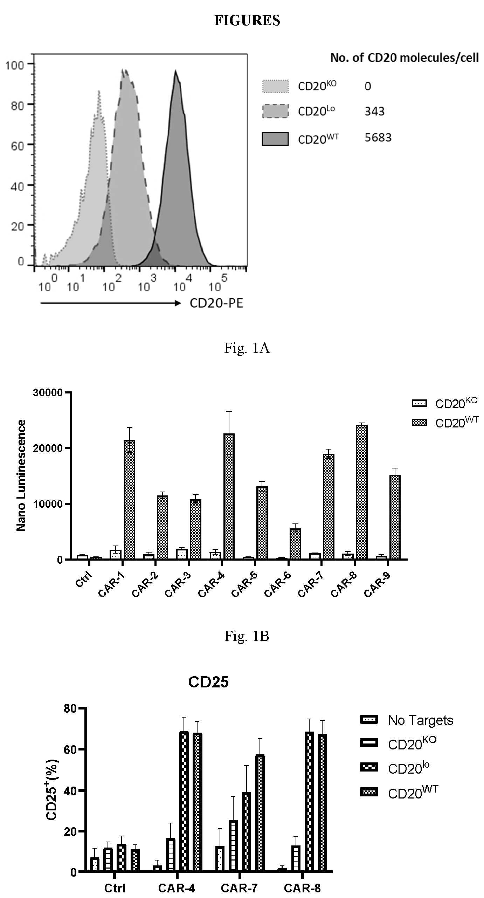

Raji cells were purchased from ATCC (Cat No.: CCL-86). CD20^{KO} Raji cells were generated by knockout of MS4A1 (gene encoding CD20) through CRISPR-Cas9 mediated gene editing. Different constructs were transduced into CD20^{KO} Raji cells to generate CD20^{lo} Raji cells (Fig. 1A).

Jurkat 76 cell line (Biovector, Cat#: 921816) was engineered to express a nanoluciferase under the control of a human IL-2 promoter. Nucleic acid molecules encoding each fusion protein were individually cloned into a lentiviral vector and transduced into this IL-2 luciferase reporter Jurkat cell line. The transduced Jurkat cells were then co-cultured with 2 different Raji cells (CD20^{KO}/CD20^{WT}) at the E:T ratio of 5:1. After 24 hours, the luciferase activity in the supernatant was measured by PE Envision multimode plate reader.

Jurkat cells transduced with CAR-4, CAR-7 and CAR-8 showed higher luciferase activity when co-cultured with CD20-expressing Raji cells, which indicates higher activation levels (Fig. 1B). CAR-8 with the CD28 hinge induced strongest activation among CARs containing Ofa-scfv, while CARs with an IgG4 hinge (CAR1 and CAR4) displayed superior activation signals among CARs containing Obin-scFv or RTX-scFv.

### Example 2: Generation and assessment of CD20 CARs in primary human T cells

Primary human T cell populations expressing the various CARs were generated. T cells, isolated from human PBMC samples obtained from healthy donors, were stimulated with CD3/CD28 beads (DYNABEAD^{®}). Three days after the stimulation, the cells were transduced with lentiviral vector comprising the polynucleotides encoding the fusion protein CAR-4, CAR-7 and CAR-8. Transduction efficiency was determined by EGFR staining 7 days post transduction. Transduced T cells were co-cultured with 3 different Raji cells (CD20^{WT}/CD20^{lo}/CD20^{KO}) at the E:T ratio of 2:1. After 48 hours, the cells were isolated and stained with CD3, EGFR, CD25, CD69 and Ki67.

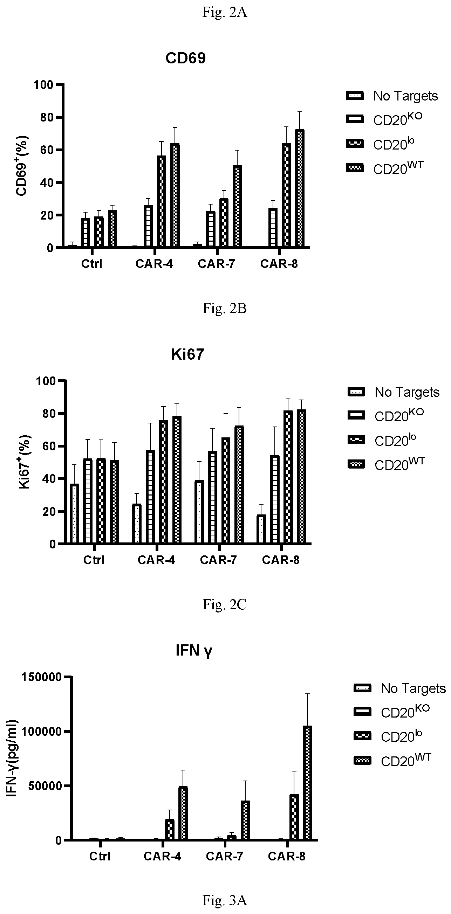

In Fig. 2A-2B, CAR-4 and CAR-8 showed higher expression of the T cell activation marker CD25 and CD69 compared to CAR-7 T cells. This is particularly evident when challenged with CD20^{lo} Raji cells. Similarly, CAR-4 and CAR-8 showed slight improvements over CAR-7 T cells when challenged with the CD20^{lo} Raji cells and stained for the proliferation marker Ki67 (Fig. 2C).

### Example 3: Cytokine release post target cell stimulation

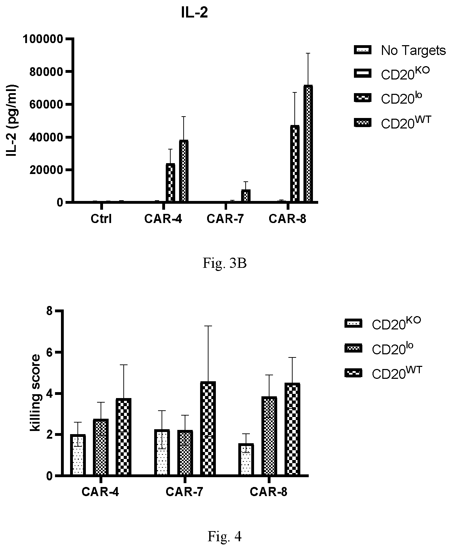

The effector cells of Example 2 were used to assess the cytokine release. The supernatants in Example 2 were collected and analyzed for accumulated IFN-γ and IL-2 cytokines. The results are depicted in Fig. 3A-3B. Cytokine secretion is positively correlated with the antigen expression. T cells transduced with CAR-8 showed the highest secretion levels of IFN-γ and IL-2 after co-cultured with either the wild-type or low expressing CD20 tumor cell lines. In contrast, CAR 4 had lower secretion and CAR 7 had near background secretion of cytokines when challenged with these low expressing targets.

### Example 4: Killing efficiency of CD20 CAR constructs

Primary human T-cells from 4 healthy donors were transduced with lentiviral vectors comprising the polynucleotides encoding the fusion protein CAR-4, CAR-7 and CAR-8 in Example 2. Transduced T cells co-cultured with 3 different Raji cells, which were pre-stained with CellTrace Far Red reagent, at the E:T ratio of 2:1. CellEvent Caspase3/7 Green Detection Reagent was added to the cell mixture to monitor the apoptosis of target cells. The apoptosis cell signal was measured by Incucyte Live-cell Analysis System SX5 for 24 hours with every 4 hours interval.

The killing score of each CAR-T was calculated as the ratio of Caspase3/7 signal from target cell group to the signal from CAR-T alone group. CAR-8 showed superior kill scores when challenged with CD20^{lo} Raji cells compared to CAR-4 (Fig. 4). The CAR-7 killing score when co-cultured with the CD20^{lo} Raji targets was similar to the background CD20^{KO} control levels. CAR8 T cells also displayed a slightly lower non-specific killing score on CD20^{KO} Raji cell compared to CAR-4 and CAR-7 (Fig. 4).

## Claims

1. A fusion protein comprising an extracellular antigen-binding domain, a transmembrane domain, a spacer located between the extracellular antigen-binding domain and the transmembrane domain and an intracellular signaling domain, wherein:
a) the extracellular antigen-binding domain is capable of specifically binding to the same epitope on CD20 as a reference antibody that comprises the amino acid sequence of SEQ ID NO: 1, SEQ ID NO: 2 or SEQ ID NO: 3;
b) the spacer consists of a hinge domain from CD28 or a hinge domain from an immunoglobulin, wherein the spacer consisting of the hinge domain from the immunoglobulin is at 12, 13, 14 or 15 amino acids in length;
c) the transmembrane domain comprises a transmembrane domain from CD28; and
d) the intracellular signaling domain comprises a primary intracellular signaling domain and a co-stimulatory signaling domain located between the transmembrane domain and the primary intracellular signaling domain, wherein the co-stimulatory signaling domain comprises a signaling domain of CD137, the primary intracellular signaling domain comprises a cytoplasmic signaling domain of CD3ζ.

2. The fusion protein of claim 1, wherein the extracellular antigen-binding domain comprises a heavy chain variable region (VH) and a light chain variable region (VL), wherein:
the VH comprises a HCDR1, a HCDR2 and a HCDR3 comprising the amino acid sequences of SEQ ID NO: 6, SEQ ID NO: 7 and SEQ ID NO: 8, respectively; the VL comprises a LCDR1, a LCDR2 and a LCDR3 comprising the amino acid sequences of SEQ ID NO: 9, SEQ ID NO: 10 and SEQ ID NO: 11, respectively; or
the VH comprises a HCDR1, a HCDR2 and a HCDR3 comprising the amino acid sequences of SEQ ID NO: 14, SEQ ID NO: 15 and SEQ ID NO: 16, respectively; the VL comprises a LCDR1, a LCDR2 and a LCDR3 comprising the amino acid sequences of SEQ ID NO: 17, SEQ ID NO: 18 and SEQ ID NO: 19, respectively; or
the VH comprises a HCDR1, a HCDR2 and a HCDR3 comprising the amino acid sequences of SEQ ID NO: 22, SEQ ID NO: 23 and SEQ ID NO: 24, respectively; the VL comprises a LCDR1, a LCDR2 and a LCDR3 comprising the amino acid sequences of SEQ ID NO: 25, SEQ ID NO: 26 and SEQ ID NO: 27, respectively;
preferably, the VH comprises an amino acid sequence having at least 90% sequence identity to SEQ ID NO: 4; the VL comprises an amino acid sequence having at least 90% sequence identity to SEQ ID NO: 5; or
the VH comprises an amino acid sequence having at least 90% sequence identity to SEQ ID NO: 12; the VL comprises an amino acid sequence having at least 90% sequence identity to SEQ ID NO: 13; or
the VH comprises an amino acid sequence having at least 90% sequence identity to SEQ ID NO: 20; the VL comprises an amino acid sequence having at least 90% sequence identity to SEQ ID NO: 21;
more preferably, the VH comprises the amino acid sequence of SEQ ID NO: 4; the VL comprises the amino acid sequence of SEQ ID NO: 5; or
the VH comprises the amino acid sequence of SEQ ID NO: 12; the VL comprises the amino acid sequence of SEQ ID NO: 13; or
the VH comprises the amino acid sequence of SEQ ID NO: 20; the VL comprises the amino acid sequence of SEQ ID NO: 21.

3. The fusion protein of claim 1 or 2, wherein the extracellular antigen-binding domain is an scFv;
preferably, the VH is fused by its C-terminus via a flexible polypeptide linker to the N-terminus of the VL;
more preferably, the extracellular antigen-binding domain comprises the amino acid sequence of SEQ ID NO: 1, SEQ ID NO: 2 or SEQ ID NO: 3.

4. The fusion protein of any one of claims 1 to 3, wherein the spacer consists of the hinge domain from CD28;
preferably, the spacer consists of the amino acid sequence of SEQ ID NO: 30.

5. The fusion protein of any one of claims 1 to 3, wherein the spacer consists of the hinge domain from IgG4, wherein the spacer consisting of the hinge domain from IgG4 is at 12 amino acids in length;
preferably, the spacer consists of the amino acid sequence of SEQ ID NO: 31.

6. The fusion protein of any one of claims 1 to 5, wherein the transmembrane domain comprises the amino acid sequence of SEQ ID NO: 33.

7. The fusion protein of any one of claims 1 to 6, wherein the primary intracellular signaling domain comprises the amino acid sequence of SEQ ID NO: 35.

8. The fusion protein of any one of claims 1 to 7, wherein the co-stimulatory signaling domain comprises the amino acid sequence of SEQ ID NO: 34.

9. The fusion protein of any one of claims 1 to 4 and 6 to 8, wherein:
a) the extracellular antigen-binding domain is capable of specifically binding to the same epitope on CD20 as a reference antibody that comprises the amino acid sequence of SEQ ID NO: 1;
b) the spacer consists of a hinge domain from CD28;
c) the transmembrane domain comprises a transmembrane domain from CD28; and
d) the intracellular signaling domain comprises a signaling domain of CD137 and a cytoplasmic signaling domain of CD3ζ;
preferably,
a) the extracellular antigen-binding domain comprises a VH comprising a HCDR1, a HCDR2 and a HCDR3 comprising the amino acid sequences of SEQ ID NO: 6, SEQ ID NO: 7 and SEQ ID NO: 8, respectively, and a VL comprising a LCDR1, a LCDR2 and a LCDR3 comprising the amino acid sequences of SEQ ID NO: 9, SEQ ID NO: 10 and SEQ ID NO: 11, respectively;
b) the spacer consists of the amino acid sequence of SEQ ID NO: 30;
c) the transmembrane domain comprises the amino acid sequence of SEQ ID NO: 33; and
d) the intracellular signaling domain comprises the amino acid sequence of SEQ ID NO: 34 and SEQ ID NO: 35;
more preferably, the fusion protein comprises the amino acid sequence of SEQ ID NO: 36.

10. The fusion protein of any one of claims 1 to 3 and 5 to 8, wherein:
a) the extracellular antigen-binding domain is capable of specifically binding to the same epitope on CD20 as a reference antibody that comprises the amino acid sequence of SEQ ID NO: 1 or SEQ ID NO: 2;
b) the spacer consists of a hinge domain from the IgG4, wherein the spacer consisting of the hinge domain from IgG4 is at 12 amino acids in length;
c) the transmembrane domain comprises a transmembrane domain from CD28; and
d) the intracellular signaling domain comprises a signaling domain of CD137 and a cytoplasmic signaling domain of CD3ζ;
preferably,
a) the extracellular antigen-binding domain comprises: a VH comprising a HCDR1, a HCDR2 and a HCDR3 comprising the amino acid sequences of SEQ ID NO: 6, SEQ ID NO: 7 and SEQ ID NO: 8, respectively, and a VL comprising a LCDR1, a LCDR2 and a LCDR3 comprising the amino acid sequences of SEQ ID NO: 9, SEQ ID NO: 10 and SEQ ID NO: 11, respectively; or
a VH comprising a HCDR1, a HCDR2 and a HCDR3 comprising the amino acid sequences of SEQ ID NO: 14, SEQ ID NO: 15 and SEQ ID NO: 16, respectively, and a VL comprising a LCDR1, a LCDR2 and a LCDR3 comprising the amino acid sequences of SEQ ID NO: 17, SEQ ID NO: 18 and SEQ ID NO: 19, respectively;
b) the spacer consists of the amino acid sequence of SEQ ID NO: 31;
c) the transmembrane domain comprises the amino acid sequence of SEQ ID NO: 33; and
d) the intracellular signaling domain comprises the amino acid sequence of SEQ ID NO: 34 and SEQ ID NO: 35;
more preferably, the fusion protein comprises the amino acid sequence of SEQ ID NO: 37 or SEQ ID NO: 38.

11. An isolated nucleic acid encoding the fusion protein of any one of claims 1-10.

12. A vector comprising the nucleic acid of claim 11, preferably, the vector is a viral vector, more preferable, the viral vector is a retroviral, lentiviral, adenoviral, or adeno-associated viral vector.

13. A cell comprising the fusion protein of any one of claims 1-10, the isolated nucleic acid of claim 11, or the vector of claims 12; preferably, the cell is a lymphocyte; more preferable, the cell is a NK cell or a T cell.

14. A pharmaceutical formulation comprising the fusion protein of any one of claims 1-10, the isolated nucleic acid of claim 11, the vector of claims 12 or the cell of claim 13 and a pharmaceutically acceptable carrier.

15. A method of treating a disease or disorder associated with CD20 in a subject, comprising administering the fusion protein of any one of claims 1-10, the isolated nucleic acid of claim 11, the vector of claims 12, the cell of claim 13 or the pharmaceutical formulation of claim 14 to the subject; optionally the subject has a relapsed or refractory disease or disorder associated with CD20 or has been previously treated with at least one regimen comprising a medicament directing against CD20;
preferably, the disease or disorder is a cancer or an autoimmune disease;
more preferably, the cancer is selected from the group consisting of leukemia, lymphoma, lung cancer, liver cancer, melanoma, breast cancer, prostate cancer, colon cancer, renal cell carcinoma, ovarian cancer, neuroblastoma and rhabdomyosarcoma; the autoimmune disease is selected from the group consisting of rheumatoid arthritis, systemic lupus erythematosus, multiple sclerosis, Wegener's disease, inflammatory bowel disease, ulcerative colitis, idiopathic thrombocytopenic purpura, thrombotic thrombocytopenic purpura, autoimmune thrombocytopenia, psoriasis, IgA nephropathy, IgM polyneuropathies, myasthenia gravis, ANCA associated vasculitis, diabetes mellitus, Reynaud's syndrome, Sjogren's syndrome, Neuromyelitis Optica and glomerulonephritis.
